# EUROPEAN PATENT APPLICATION

(11) **EP 4 414 710 A1**
(43) Date of publication of application: **14.08.2024**
(21) Application number: 22878167.0
(22) Date of filing: 19.07.2022
(51) Int. Cl.: G01N 35/00

(54) **SPECIMEN INFORMATION SETTING METHOD, SPECIMEN INFORMATION SETTING PROGRAM, AND SPECIMEN INFORMATION SETTING DEVICE**

(30) Priority: 04.10.2021 JP 2021163529
(71) Applicant: Shimadzu Corporation, Nakagyo-ku, Kyoto-shi, Kyoto 604-8511 (JP)
(72) Inventor: SHIMIZU, Kazunori, Kyoto-shi, Kyoto 604-8511 (JP)
(74) Representative: Müller-Boré & Partner Patentanwälte PartG mbB
(86) International application number: PCT/JP2022/027978
(87) International publication number: WO 2023/058296

(57) **Abstract**

A sample information setting method for setting information on a sample according to an operator's operation in an injection device (sample injection device (10)) for injecting a sample into each of a plurality of injection targets (wells), includes a step (S0) of displaying a plurality of first images (well images (7)) corresponding to the plurality of injection targets (wells), respectively; a step (S4) of displaying a second image (first menu image (721), third menu image (723)) showing, as a menu, information on at least one sample (first selection option image (73), second selection option image (74), third selection option image (75)) capable of being set to the injection target (well) corresponding to the first image (well image (7)) included in a region within an array (well array image (70)) of the plurality of injection targets (wells) displayed by the plurality of the first images (well images (7)), in response to completion of a first operation (dragging operation) of selecting the region by a dragging operation, the region including the first image (well image (7)) of the injection target (well); and a step (S53) of setting the information on the sample selected by a second operation (selection option selection operation) from the information on the at least one sample included in the second image (first menu image (721), third menu image (723)) to the injection target (well) corresponding to the first image (well image (7)) included in the region.

## Description

### Technical Field

The present invention relates to a sample information setting method, a sample information setting program, and a sample information setting device.

### Background Art

In various tests and analyses, such as a chemical test and a biological analysis, a sample injection device that injects a sample into each of a plurality of injection targets, such as a plurality of wells in a well plate, for example, is used. The setting of what kind of sample is to be injected in a sample injection device can be performed, for example, by software for setting information on the sample to be injected.

For example, as a method for setting information on a sample to be injected into multiple injection targets, there is a method in which a template image having a cell arrangement corresponding to the well arrangement is generated and displayed on a display unit, and an analysis operator inputs information into each cell by direct text input or by drag and drop operations from a sample list generate in advance (Patent Document 1).

### Prior Art Document

### Patent Document

Patent Document 1: Japanese Unexamined Patent Application Publication No. 2019-74441

### SUMMARY OF THE INVENTION

### Problems to be Solved by the Invention

However, a conventional sample information setting method, such as the method described in Patent Document 1, has a problem that operations, such as direct text input operations and drag-and-drop operations from a pre-generated sample list, are complicated.

The purpose of the present invention is to simplify the operation when setting a sample to be injected into multiple injection targets.

### Means for Solving the Problems

According to an aspect of the present disclosure, a sample information setting method of setting sample information according to an operator's operation in an injection device for injecting a sample into each of a plurality of injection targets, includes:
a step of displaying a plurality of first images corresponding to the plurality of injection targets, respectively;
a step of displaying a second image showing, as a menu, information on at least one sample capable of being set to the injection target corresponding to the first image included in a region within an array of the plurality of injection targets displayed by the plurality of the first images, in response to completion of a first operation of selecting the region by a dragging operation, the region including the first image of the injection target; and
a step of setting the information on the sample selected by a second operation from the information on the at least one sample included in the second image to the injection target corresponding to the first image included in the region.

According to another aspect of the present disclosure, a sample information setting program for making a computer execute processing to set information on a sample according to an operator's operation in an injection device for injecting a sample into each of a plurality of injection targets, the processing comprises:
a step of displaying a plurality of first images corresponding to the plurality of injection targets, respectively;
a step of displaying a second image showing, as a menu, information on at least one sample capable of being set to the injection target corresponding to the first image included in a region within an array of the plurality of injection targets displayed by the plurality of the first images, in response to completion of a first operation of selecting the region by a dragging operation, the region including the first image of the injection target; and
a step of setting information on the sample selected by a second operation from the information on the at least one sample included in the second image to the injection target corresponding to the first image included in the region.

According to another aspect of the present disclosure, a sample information setting device for setting information on a sample according to an operator's operation in an injection device for injecting a sample into each of a plurality of injection targets, the device includes:
a display unit;
a controller; and
a storage unit,
wherein the controller executes
first image display processing for displaying a plurality of first images corresponding to the plurality of injection targets, respectively, on the display unit,
second image display processing for displaying a second image showing, as a menu, information on at least one sample capable of being set to the injection target corresponding to the first image included in a region within an array of the plurality of injection targets displayed by the plurality of the first images, in response to completion of a first operation of selecting the region by a dragging operation, the region including the first image of the injection target, and
setting processing for causing the storage unit to store data for setting the information on the sample selected by a second operation from the information on the at least one sample included in the second image to the injection target corresponding to the first image included in the region.

### Effects of the Invention

In accordance with one aspect of the present disclosure, in response to the completion of the first operation of selecting the region that includes the target first image by a dragging operation within the array of multiple injection targets displayed by multiple first images, the second image is displayed, showing via a menu the information on at least one sample capable of being set to the injection target corresponding to the first image within the region. Therefore, the first operation serves as both the selection operation of the region including the first image to be set and the display operation of the second image showing the information on the sample capable of being set as the injection target corresponding to the first image in the menu. Further, the information on the sample capable of being set to the injection target corresponding to the first image included in the region selected by the first operation is shown in the menu by the second image. In addition, the information on the sample selected by the second operation from the information on the sample included in the second image is set to the injection target corresponding to the first image included in the region. As a result, it is possible to simplify the operation for making the same setting for multiple injection targets.

In accordance with another aspect of the present disclosure, in response to the completion of the first operation of selecting the region that includes the target first image by a dragging operation within the array of multiple injection targets displayed by multiple first images, the second image is displayed, showing via a menu the information on at least one sample capable of being set to the injection target corresponding to the first image within the region. Therefore, the first operation serves as both the selection operation of the region including the first image to be set and the display operation of the second image showing the information on the sample capable of being set as the injection target corresponding to the first image in the menu. For this reason, it is possible to provide a sample information setting program that simplifies operations when setting a sample to be injected into multiple injection targets. It also facilitates the process of making the same setting for multiple injection targets. Further, the information on the sample capable of being set to the injection target corresponding to the first image included in the region selected by the first operation is shown in the menu by the second image. In addition, the information on the sample selected by the second operation from the information on the sample included in the second image is set to the injection target corresponding to the first image included in the region. As a result, it is possible to simplify the operation for making the same setting for multiple injection targets.

According to still another aspect of the present disclosure, in response to the completion of the first operation of selecting the region that includes the target first image by a dragging operation within the array of multiple injection targets displayed by multiple first images, the second image is displayed, showing via a menu the information on at least one sample capable of being set to the injection target corresponding to the first image within the region. Therefore, the first operation serves as both the selection operation of the region including the first image to be set and the display operation of the second image showing the information on the sample capable of being set as the injection target corresponding to the first image in the menu. Therefore, it is possible to provide a sample information setting device capable of simplifying the operations when setting samples to be injected into multiple injection targets. It also facilitates the process of making the same setting for multiple injection targets. Further, the information on the sample capable of being set to the injection target corresponding to the first image included in the region selected by the first operation is shown in the menu by the second image. In addition, the information on the sample selected by the second operation from the information on the sample included in the second image is set to the injection target corresponding to the first image included in the region. As a result, it is possible to simplify the operation for making the same setting for multiple injection targets.

### BRIEF DESCRIPTION OF THE DRAWINGS

FIG. 1 is a diagram showing a configuration of a well information setting system according to a first embodiment.
FIG. 2 is a block diagram showing a main configuration of a tablet terminal 1.
FIG. 3 is a diagram showing a well array image 70 displayed on a display unit 3 according to the first embodiment.
FIG. 4 is a diagram showing a well information setting method for well images 7 in a first well displayed on the display unit 3.
FIG. 5 is a diagram showing a well information deletion method for well images 7 in the first well displayed on the display unit 3.
FIG. 6 is a diagram showing a well information deletion method for well images 7 in the first well displayed on the display unit 3.
FIG. 7 is a diagram showing a well information addition setting method for well images 7 in a first well displayed on the display unit 3.
FIG. 8 is a diagram showing a well information setting method for well images 7 in a second well displayed on the display unit 3.
FIG. 9 is a diagram showing a multiple well information setting method for well images 7 in a second well displayed on the display unit 3.
FIG. 10 is a diagram showing a multiple well information setting method for well images 7 in second well displayed on the display unit 3.
FIG. 11 is a diagram showing a well information deletion method in well images 7 for the second well displayed on the display unit 3.
FIG. 12 is a diagram showing a well information deletion method for well images 7 in the second well displayed on the display unit 3.
FIG. 13 is a flowchart showing well selection processing.
FIG. 14 is a flowchart showing menu image display processing.
FIG. 15 is a flowchart showing well information setting processing.
FIG. 16 is a diagram showing a well array image 70 displayed on a display unit 3 according to a second embodiment.
FIG. 17 is a diagram showing a well information setting method for well images 7 in a first well and a second well displayed on the display unit 3.
FIG. 18 is a diagram showing a well information setting method for well images 7 in a first well and a second well displayed on the display unit 3.
FIG. 19 is a diagram showing a well information deletion method for well images 7 in the second well displayed on the display unit 3.
FIG. 20 is a diagram showing a well information deletion method for well images 7 in the second well displayed on the display unit 3.
FIG. 21 is a diagram showing a well information deletion method for well images 7 in the second well displayed on the display unit 3.
FIG. 22 is a diagram showing a well array image 70 displayed on a display unit 3 according to a third embodiment.
FIG. 23 is a diagram showing a well information setting method for well images 7 in a first well displayed on the display unit 3.
FIG. 24 is a diagram showing a well information setting method for well images 7 in a first well displayed on the display unit 3.

### EMBODIMENTS FOR CARRYING OUT THE INVENTION

Hereinafter, referring to the drawings, some embodiments of this disclosure will be described.

### [First Embodiments]

### [Configuration of Well Information Setting System]

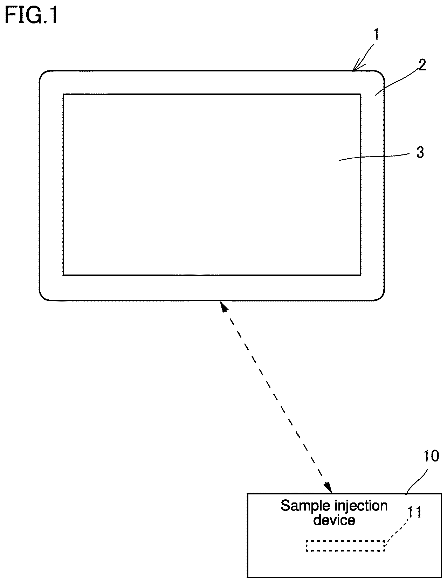

FIG. 1 is a diagram showing a configuration of a well information setting system according to a first embodiment.

Referring to FIG. 1, the well information setting system, which is a sample information setting system, includes a tablet terminal 1 and a sample injection device 10. The well information setting system is a system for setting the information (hereinafter referred to as "well information") on a sample to be injected by the sample injection device 10, which possesses the function of injecting a sample into a plurality of wells provided in the well plate 11, according to the operation of the tablet terminal 1 by the operator.

The sample injection device 10 is a device for injecting a sample into each of the plurality of wells that serves as injection targets on the well plate 11. The tablet terminal 1 is a device that can set well information, which is the information on a sample to be injected into each of the plurality of wells in the sample injection device 10.

The tablet terminal 1 is capable of performing data communication with the sample injection device 10 wirelessly. The tablet terminal 1 is a terminal device that the operator can carry. The tablet terminal 1 includes a display unit 3 including a touch panel and a frame 2. The frame is a case that protects regions in the tablet terminal 1 other than the region where the display unit 3 is provided. The operator can perform touch operations such as tapping and dragging with their finger on the display unit 3.

The sample injection device 10 may be a device incorporated into a system of an analysis device that analyzes samples and operates as a sample injection unit, or it may be a stand-alone device that injects a sample. The tablet terminal 1 may be a dedicated terminal device for a well information setting system or a general-purpose terminal device.

### [Configuration of Tablet Terminal 1]

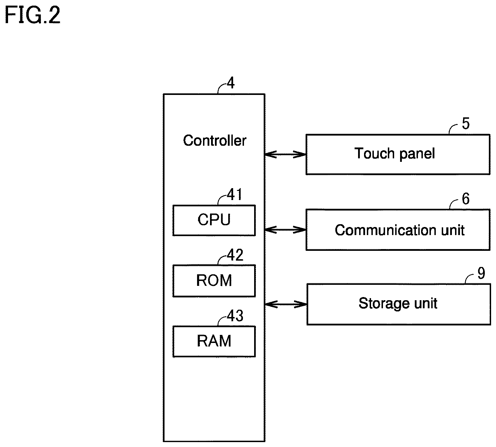

FIG. 2 is a block diagram showing the main configuration of the tablet terminal 1.

Referring to FIG. 2, the tablet terminal 1 includes a controller 4, a touch panel 5, a communication unit 6, and a storage unit 9. The communication unit 6 is used for wireless and wired communication between the tablet terminal 1 and the sample injection device 10.

The controller 4 is configured by a computer comprising a CPU (Central Processing Unit) 41, a ROM (Read Only Memory) 42, and a RAM (Random Access Memory) 43. The ROM 42 stores data such as programs to be executed by the CPU 41. The RAM 43 is used as a work area when executing a program. The CPU 41 executes a program read from the ROM 42 or the storage unit 9 into the RAM 43.

The touch panel 5 is a touch panel that combines an image display unit comprising an LCD panel and an operation detection unit comprising a touch sensor. The touch panel 5 displays various images on the image display unit based on the image display signal provided by the controller 4, and detects the operator's touch operation through the operation detection unit, sending the operation detection signal to the controller 4.

The communication unit 6 enables data communication via wireless communication with the communication unit installed in the sample injection device 10. In the case where the tablet terminal 1 is connected to the sample injection device 10 with a communication cable, the communication unit 6 enables data communication with the communication unit provided in the sample injection device 10 by wired communication. The storage unit 9 is composed of an HDD (Hard Disk Drive) or an SSD (Solid State Drive). The storage unit 9 stores information, such as various application programs and various data.

### [Well Information Setting Method]

Next, the well information setting method using the tablet terminal 1 will be described.

### (Well Array Image)

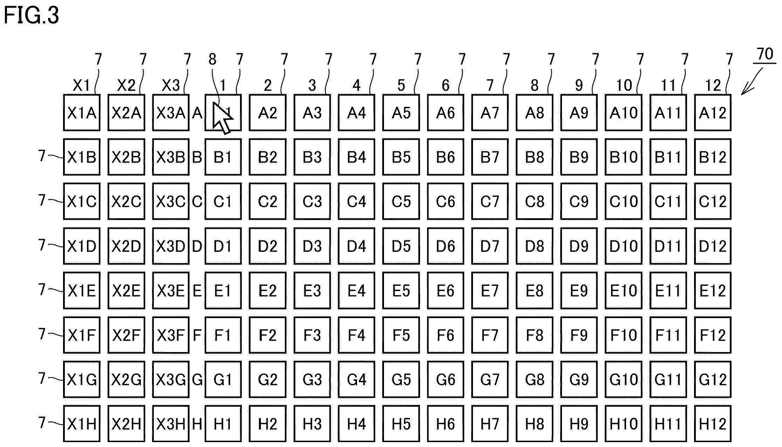

FIG. 3 is a diagram showing a well array image 70 displayed on the display unit 3 by the first display unit.

The well array image 70 is an image that mimics the array of wells when the actual plurality of wells provided on the well plate 11 is viewed in plan view. In the well array image 70, well images 7, each correlating one well provided on the well plate 11 with one well image, are displayed corresponding to the actual well array. Each well image 7 is a rectangular image that mimics an actual well. In each well image 7, a well symbol that can identify the position of the arrayed well is displayed. Well symbols are, for example, symbols such as A1, A2, ..., H12, as well as X1A, X2A, ..., X3H, as shown in FIG. 3.

In the drawings that show the well array image 70, as shown in FIG. 3, etc., in order to make it easier to explain the position being operated by the operator, the touch position 8 that the operator is currently touching is indicated by a white-outlined arrow.

The group of a total of 96 well images 7 consisting of 8 rows (rows indicated by "A" to "H" in the figure) x 12 columns (columns indicated by "1" to "12" in the figure) on the right side in the well array image 70 is a group of well images 7 corresponding to a first well, called "96 wells," provided in the well plate 11. The group of a total of 24 well images 7, consisting of 8 rows (rows indicated by "A" to "H" in the figure) x 3 columns (columns indicated by "X1" to "X3" in the figure) on the left side in the well array image 70, is a group of well images 7 corresponding to a second well, called "extra stand wells," provided in the well plate 11.

In the group of well images 7 in the first well, well symbols of 96 types ranging from A1 to H12 are shown. In the group of well images 7 in the second well, well symbols of 24 types ranging from X1A to X3H are shown.

As will be described below, all well images 7 can display the setting information (well information) for the sample to be injected into the corresponding well as an image (set image 77).

### (Well Information Setting Method for Well Images 7 in First Well)

Next, the well information setting method for well images 7 in the first well will be described. One feature of the well information setting method described below is as follows. That is, an operator selects a region displaying well images 7 with unset well information by touching and dragging their finger on the display unit 3. Upon releasing the finger from the display unit 3, thereby ending the series of drag operations, a first menu image 721 is displayed. This menu image 721 shows selection options for the sample to be set in the wells within the selected region. More specifically, when a dragging operation, initiated by touching a finger on the display unit 3, is detected by the operation detection unit of the touch panel 5, an operation detection signal is transmitted from the operation detection unit to the controller 4. When the controller 4 receives the detection signal of such a dragging operation, it performs control to display the first menu image 721 on the display unit 3.

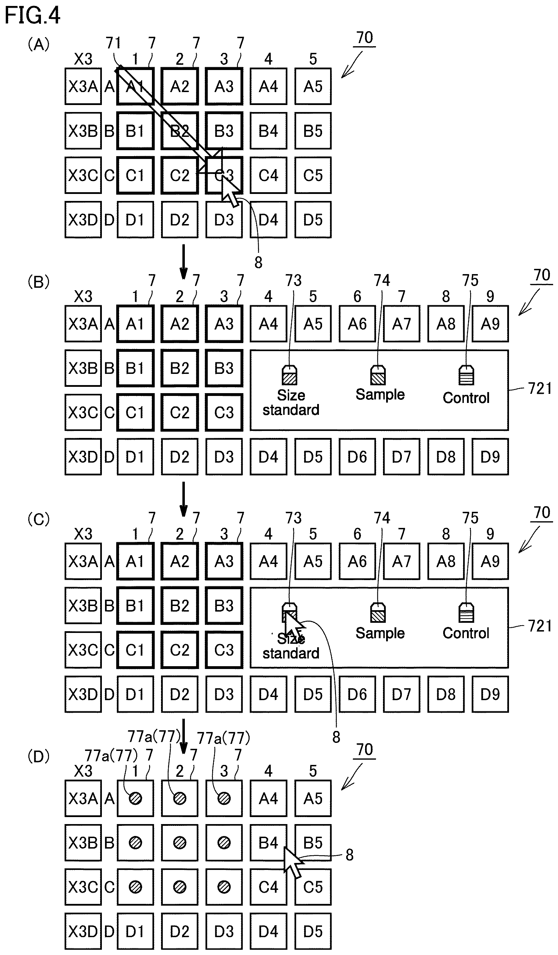

FIG. 4 is a diagram showing the well information setting method for the well images 7 in the first well displayed on the display unit 3. FIG. 4(A) to FIG. 4(D) show the display state of the well array image 70, which changes in response to operations. In FIG. 4, although all of the well images 7 shown in FIG. 3 are actually displayed on the display unit 3, only the minimum number of well images 7 necessary for explanation are shown due to the limitations of the drawing's range.

As shown in FIG. 4(A), it is assumed that an operator performs the following dragging operation on the display unit 3 to select wells for setting well information in a state in which the well array image 70, with well information not yet set, is displayed. The assumed dragging operation is an operation in which the operator touches their finger to the display unit 3 and, for example, drags in the direction indicated by the arrow 71 in the figure from the well image 7 at "A1" to the well image 7 at "C3". This selects a region that includes the well images 7 corresponding to the setting target wells.

In response to the detection of the dragging operation shown in FIG. 4(A), the well images 7 that fall within the range of the rows and columns covered by the dragging operation in the well array are selected. As a result, the actual wells corresponding to the well images 7 that fall within the range of the rows and columns covered by the dragging operation are selected as targets for setting well information. Such a dragging operation is also called a well selection operation. The well images 7 selected through such a dragging operation are indicated in such a manner that they can be distinguished from the well images 7 that are not selected, by highlighting the images (hereinafter referred to as "highlighting"), as shown by the thick-lined frame in the figure.

After performing the dragging operation shown in FIG. 4(A), when the operator releases their finger from the display unit 3, thereby releasing the touch state, the completion of the dragging operation is detected. Upon detection of the completion of the dragging operation, as illustrated in FIG. 4(B), a first menu image 721 displaying sample selection options of three types, "Size Standard," "Sample," and "Control," is displayed on the right side of the screen with the position where the finger was released as the reference point. In the first menu image 721, three selection option images are displayed: the first selection option image 73 for "Size Standard" sample, the second selection option image 74 for "Sample," and the third selection option image 75 for "Control." Each of the first selection option image 73, the second selection option image 74, and the third selection option image 75 includes an image of a container containing a sample and an image indicating the name of the sample type are displayed, with the color of each container being different. The first menu image 721 can be displayed at any position provided that it is near the position where the dragging operation ended. As described above, by displaying the first menu image 721 near the position where the dragging operation ended, it becomes easier to visually recognize the first menu image 721 after the completion of the dragging operation.

As shown in FIG. 4(C), with the first menu image 721 displayed, when the operator performs, for example, a touch operation on the first selection option image 73, and the touch operation is detected, the selection option for the first selection option image 73 becomes selected. When the operation to select the selection option for the first selection option image 73 is performed, as illustrated in FIG. 4(C), data is stored in the storage unit 9. This data is used to set the "size standard" sample, corresponding to the first selection option image 73, as the sample to be injected into the wells corresponding to the well images 7, which were selected as targets for well information settings based on the aforementioned dragging operation.

As described above, with the first menu image 721 displayed, when the operator performs a touch operation on any one of the first selection option images 73 to 75, and once the touch operation is detected, the data is stored in the storage unit 9. The data is used to set the sample type corresponding to the touched selection option image as the sample to be injected into the wells corresponding to the well images 7, which has been selected as the setting targets of the well information based on the dragging operation. The operation that the operator selects any one of the first selection option image 73 to the third selection option image 75 by performing a touch operation in a state in which the first menu image 721 is displayed is also referred to as a selection option selection operation.

As shown in FIG. 4(C), in the state in which the first menu image 721 is displayed, for example, when a touch operation of the first selection option image 73 by the operator is detected, as shown in FIG. 4(D), single set images 77a each comprising a circular image of the same color as the image color of the sample in the first selection option image that was operated are displayed as the set images 77 in the well images 7 selected as the well information setting targets. Furthermore, as shown in FIG. 4(D), the highlighting that was performed on the selected well image 7 is deactivated. This allows the operator to easily confirm which type of sample is set in which well, since the set type of sample is indicated by an image in the selected well images 7

### (Well Information Deletion Method for Well Image 7 in First Well)

Next, a method for deleting well information in the well images 7 in the first well will be described. One feature of the well information deletion method described below is as follows. That is, the operator selects a region in which both the well images 7 for which well information has been set and the well images 7 for which well information has not yet been set, in response to a dragging operation by touching their finger on the display unit 3. Thereafter, upon completion of the dragging operation by releasing their finger from the display unit 3, thereby ending the touch operation, the second menu image 722 that shows a deletion selection option image 76 for selecting deletion of the well information corresponding to the wells that have been set in the selected area.

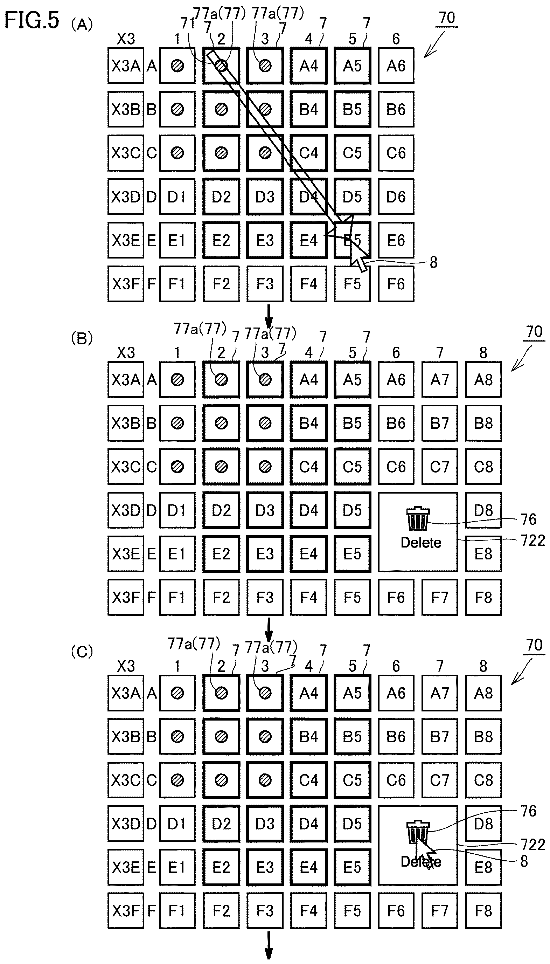

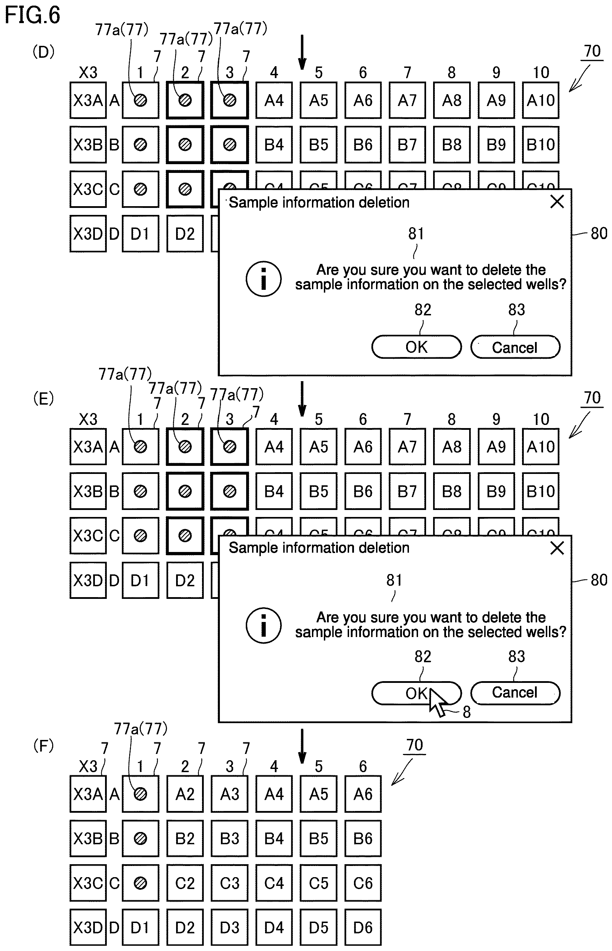

FIG. 5 and FIG. 6 show the well information deletion method for the well images 7 in the first well displayed on the display unit 3. In FIG. 5(A) to FIG. 5(C) and FIG. 6(D) to FIG. 6(F), the display state of the well array image 70, which changes in response to operations, is shown. In FIG. 5 and FIG. 6, although all of the well images 7 shown in FIG. 3 are actually displayed on the display unit 3, only the minimum number of well images 7 necessary for explanation are shown due to the limitations of the drawing's range.

As shown in FIG. 5(A), it is assumed that an operator performs the following dragging operation on the display unit 3 to select wells for setting well information in a state in which the well array image 70 is displayed. The well array image 70 depicts set images 77 comprising single set images 77a, indicating that the samples of "size standard" have been set as well information, in 9 (nine) well images of "A1" to "A3." "B1" to "B3," and "C1" to "C3." The assumed dragging operation is an operation in which the operator touches their finger to the display unit 3 and, for example, drags in the direction indicated by the arrow 71 in the figure from the well image 7 at "A2" to the well image 7 at "E5." This selects a region that includes the well images 7 corresponding to the setting target wells.

In response to the detection of the dragging operation shown in FIG. 5(A), the well images 7 that fall within the range of the rows and columns covered by the dragging operation in the well array are selected. As a result, the actual wells corresponding to the well images 7 that include the well images 7 for which well information has been set and the well images 7 for which well information has not been set, which are included within the range of rows and columns where the dragging operation was performed, are selected. The well images 7 selected by the dragging operation are indicated as being highlighted, as shown by the bold frames in the figure, allowing them to be distinguished from the well images 7 that have not been selected.

After performing the dragging operation shown in FIG. 5(A), when the operator releases their finger from the display unit 3, thereby releasing the touch state, the completion of the dragging operation is detected. When the end of the dragging operation is detected, as shown in FIG. 5(B), a second menu image 722 that displays a deletion selection option image 76 labeled "Delete" is displayed on the right side, with the position from which the finger was released as the reference point. In the deletion selection option image 76, an image of a trash container and the word "delete" are displayed. Note that the second menu image 722 can be displayed at any position as long as it is near the position where the dragging operation ended. As described above, by displaying the second menu image 722 near the position where the dragging operation ended, it becomes easier to visually recognize the second menu image 722 after the completion of the dragging operation.

As shown in FIG. 5(C), with the second menu image 722 displayed, when the operator performs a touch operation on the deletion selection option image 76 and the operation is detected, as shown in FIG. 6(D), a deletion confirmation image 80 will be displayed on the right side with the position from which the finger was released as a reference position in response to the touch operation. In the deletion confirmation image 80, messages are displayed. The messages include a message image 81 to confirm whether the sample information set in the wells corresponding to the selected well images 7 should be deleted, an OK image 82 indicating an icon for confirming the deletion, and a cancel image 83 indicating an icon for rejecting the deletion.

As shown in FIG. 6(E), with the deletion confirmation image 80 displayed, when the operator touches the OK image 82 and the touch operation is detected, the well information stored in the storage unit 9 for the wells corresponding to the well images 7 for which well information has been set among the selected well images 7 is deleted according to the touch operation. As shown in FIG. 6(F), in the selected well images 7 corresponding to the wells for which well information has been deleted out of the selected well images 7, the set images 77 consisting of the single set images 77a are deleted, and the well symbols are displayed. Further, as shown in FIG. 6(E), the highlighting that was displayed on the selected well images 7 is deactivated. This allows the operator to easily confirm the well images 7 corresponding to the wells for which well information has been deleted.

Note that for the well images 7 for which well information has already been set in the case where the well images 7 selected by the dragging operation are the images corresponding to the first well, instead of displaying the menu image for selecting the deletion of the sample information that has been set in the first well corresponding to the well images 7 as described above, it may be configured such that the information on the samples that have already been set corresponding to the first well can be changed in the form of overwriting the information on the samples selected by the second operation, as described below. For example, in the case where the well images 7 selected by the dragging operation are the images corresponding to the first wells, for the well images 7 for which well information has already been set, a menu image such as the first menu image 721, which displays selection options for the the samples to be set in the first well, is displayed to enable the selection operation for a new type of sample. Then, with such a menu image displayed, when a new sample to be set in the first well corresponding to the well image 7 for which well information has already been set is selected by a selection operation, the first well information overwrite processing may be performed to overwrite the set well information with the information on the newly selected sample. By performing such processing, the type of sample to be set can be easily changed for the first well for which well information has already been set.

### (Well Information Additional Setting Method for Well Images 7 in First Well)

Next, the well information additional setting method for the well images 7 in the first well will be described. One feature of the well information additional setting method described below is as follows. That is, an operator selects a region displaying well images 7 with unset well information by touching and dragging their finger on the display unit 3. Upon releasing the finger from the display unit 3, thereby ending the drag operation, a first menu image 721 is displayed. This first menu image 721 shows selection options for the samples to be set in the wells within the selected region.

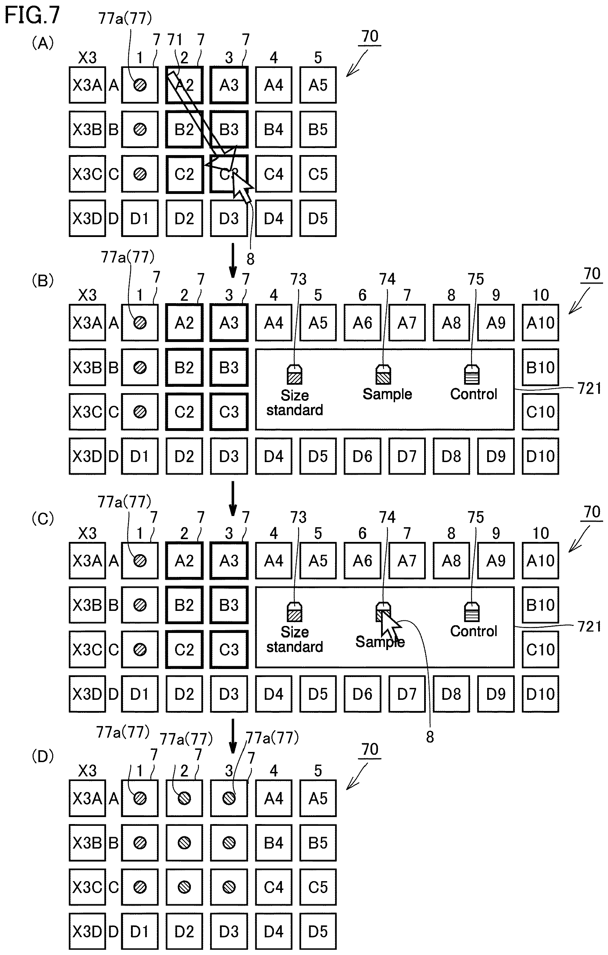

FIG. 7 is a diagram showing the well information additional setting method for the well images 7 in the first well displayed on the display unit 3. FIG. 7(A) to FIG. 7(D) show the display state of the well array image 70, which changes in response to operations. In FIG. 7, although all of the well images 7 shown in FIG. 3 are actually displayed on the display unit 3, only the minimum number of well images 7 necessary for explanation are shown due to the limitations of the drawing's range.

As shown in FIG. 7(A), it is assumed that an operator performs the following dragging operation on the display unit 3 to select wells for the additional setting of well information in a state in which the well array image 70 is displayed. The well array image 70 depicts the set images 77 comprising single set images 77a, indicating that the samples of "size standard" have been set as well information, in the 3 (three) well images of "A1," "B1," and "C1." The assumed dragging operation is an operation to select a region including the well images corresponding the wells to be additionally set by the operator contacting the display unit 3 and dragging their finger in the direction shown by the arrow 71 in the figure between the well image 7 of "A2" to the well image 7 of "C3," where the well information has not been set, for example, to select the well images 7 corresponding to the wells to be additionally set. As described above, the addition of the well information for the well images 7 in the first well is performed for the well images 7 for which well information has not yet been set.

In response to the detection of the dragging operation shown in FIG. 7(A), the well images 7 in the range of the rows and columns where the dragging operation was performed are selected. With this, the actual wells corresponding to the well images 7 within the range of the rows and columns where the dragging operation was performed are selected as the setting targets for the well information. The well images 7 selected by the dragging operation are indicated as being highlighted, as shown by the bold frames in the figure.

After performing the dragging operation shown in FIG. 7(A), when the operator releases their finger from the display unit 3, thereby releasing the touch state, the completion of the dragging operation is detected. When the end of the dragging operation is detected, as shown in FIG. 7(B), a first menu image 721 displaying a first selection option image 73, a second selection option image 74, and a third selection option image 75 is displayed.

As shown in FIG. 7(C), with the first menu image 721 displayed, when the operator performs, for example, a touch operation on the second selection option image 74, and the touch operation is detected, the selection option of the second selection option image 74 becomes selected. When the operation to select the selection option for the second selection option image 74 is performed as illustrated in FIG. 7(C), data is stored in the storage unit 9. This data is used to set the "sample," corresponding to the second selection option image 74, as the sample to be injected into the wells corresponding to the well images 7, which were selected as targets for setting well information based on the aforementioned dragging operation.

As described above, with the first menu image 721 displayed, when the operator performs a touch operation on any one of the first selection option image 73 to the third selection option image 75, and once the touch operation is detected, the data is stored in the storage unit 9. The data is used to set the sample type corresponding to the touched selection option image as the sample to be injected into the wells corresponding to the well images 7, which have been selected as the setting target of the well information based on the dragging operation.

As shown in FIG. 7(C), with the first menu image 721 displayed, for example, when a touch operation on the second selection option image 74 by the operator is detected, as shown in FIG. 7(D), a single set image 77a comprising a circular image of the same color as the image color of the sample in the touched selection option image is displayed as the set image 77 in the well image 7 selected as the well information setting target. Furthermore, as shown in FIG. 7(D), the highlighting that was performed on the selected well images 7 is deactivated. This allows the operator to easily confirm which type of sample has been set in which well, since the set type of sample is indicated by an image in the selected well images 7

### (Well Information Setting Method for Well Images 7 in Second Well)

Next, the well information setting method for well images 7 in the second well will be described. One feature of the well information setting method described below is as follows. That is, an operator selects a region displaying well images 7 with unset well information by touching and dragging their finger on the display unit 3. Upon releasing the finger from the display unit 3, thereby ending the series of drag operations, a first menu image 721 is displayed. This first menu image 721 shows selection options for the samples to be set in the wells within the selected region.

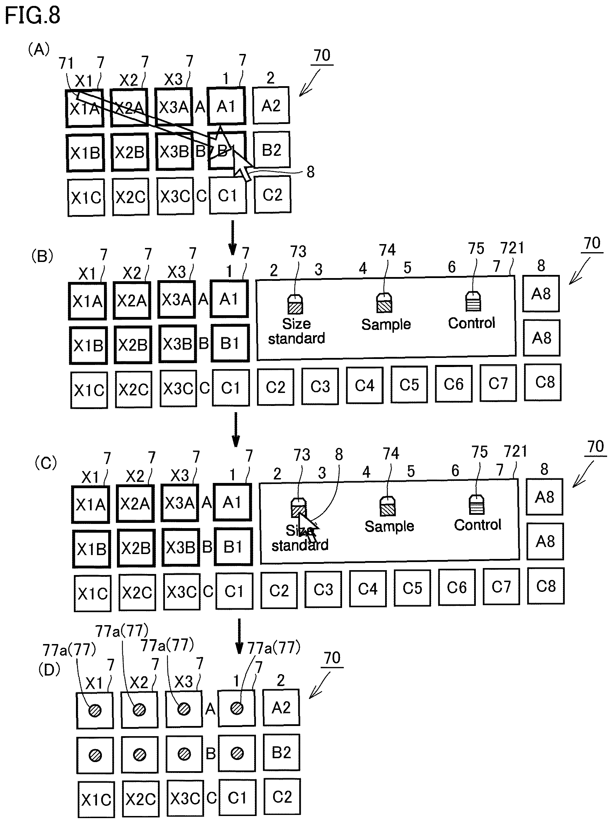

FIG. 8 is a diagram showing the well information setting method for the well images 7 in the second well displayed on the display unit 3. FIG. 8(A) to FIG. 8(D) show the display state of the well array image 70, which changes in response to operations. In FIG. 8, although all of the well images 7 shown in FIG. 3 are actually displayed on the display unit 3, only the minimum number of well images 7 necessary for explanation are shown due to the limitations of the drawing's range.

As shown in FIG. 8(A), it is assumed that an operator performs the following dragging operation on the display unit 3 to select wells for setting well information in a state in which the well array image 70, with well information not yet set, is displayed. The assumed dragging operation is an operation in which the operator touches their finger to the display unit 3 and, for example, drags in the direction indicated by the arrow 71 in the figure from the well image 7 at "X1A" to the well image 7 at "B1." This selects a region that includes the well images 7 corresponding to the setting target wells.

In response to the detection of the dragging operation shown in FIG. 8(A), the well images 7 in the first well and the second well, which fall within the range of the rows and columns covered by the dragging operation in the well array are selected. As a result, the actual wells corresponding to the well images 7 of the first well and the second well, which fall within the range of the rows and columns covered by the dragging operation, are selected as targets for setting well information. The well images 7 selected by such a dragging operation are indicated as being highlighted, as shown by the bold frames in the figure, allowing them to be distinguished from the well images 7 that have not been selected.

After performing the dragging operation shown in FIG. 8(A), when the operator releases their finger from the display unit 3, thereby releasing the touch state, the completion of the dragging operation is detected. When the end of the dragging operation is detected, as shown in FIG. 8(B), a first menu image 721 displaying a first selection option image 73, a second selection option image 74, and a third selection option image 75 is displayed.

As shown in FIG. 8(C), with the first menu image 721 displayed, when the operator performs, for example, a touch operation on the first selection option image 73, and the touch operation is detected, the selection option for the first selection option image 73 becomes selected. When the operation to select the selection option for the first selection option image 73 is performed, as illustrated in FIG. 8(C), data is stored in the storage unit 9. This data is used to set the "size standard" sample, corresponding to the first selection option image 73, as the sample to be injected into the wells corresponding to the well images 7, which were selected as targets for well information settings based on the aforementioned dragging operation.

As described above, with the first menu image 721 displayed, when the operator performs a touch operation on any one of the first selection option images 73 to 75, and once the touch operation is detected, the data is stored in the storage unit 9. The data is used to set the sample type corresponding to the touched selection option image as the sample to be injected into the wells corresponding to the first well images 7 in the first well and the second well, which have been selected as the setting target of the well information based on the dragging operation.

As shown in FIG. 8(C), when a touch operation of the first selection option image 73 by the operator, for example, is detected while the first menu image 721 is displayed, as shown in FIG. 8(D), a single set image 77a, composed of a circular image of the same color as the sample image color in the touched selection option image, is displayed as the set image 77 in the well image 7 selected as the target of well information setting. Furthermore, as shown in FIG. 8(D), the highlighting that was performed on the selected well images 7 is deactivated. This allows the operator to easily confirm which type of sample has been set in which well, since the set type of sample is indicated by an image in the selected well images 7

### (Well Information Additional Setting Method for Well Images 7 in Second Well)

Next, the well information additional setting method for the well images 7 in the second well will be described. One feature of the well information additional setting method described below is as follows. That is, an operator selects a region displaying well images 7 with unset well information by touching and dragging their finger on the display unit 3. Upon releasing the finger from the display unit 3, thereby ending the series of drag operations, a third menu image 723 is displayed. This third menu image 723 shows selection options for the samples to be set in the wells within the selected region and a deletion selection option.

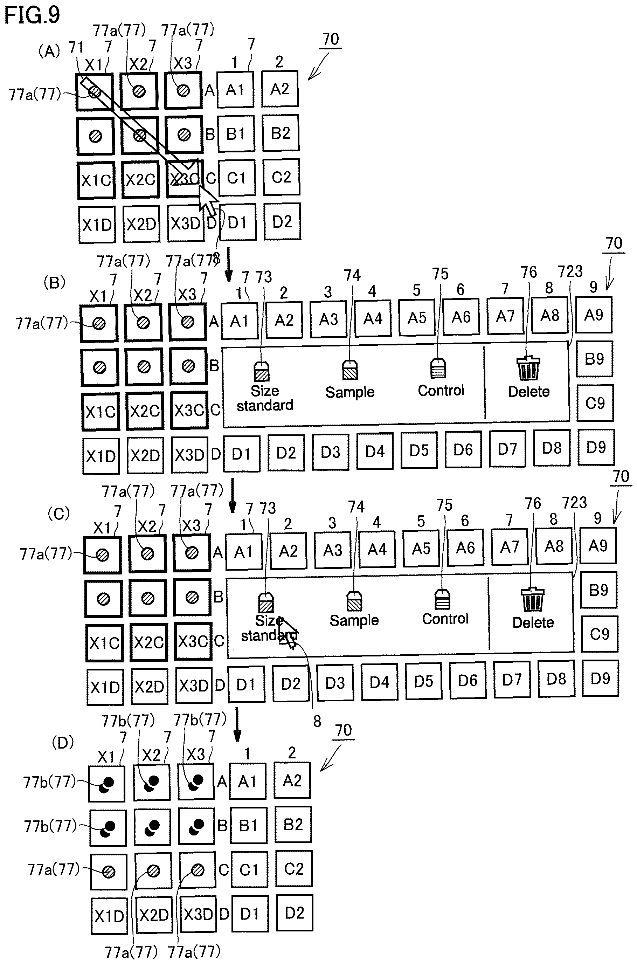

FIG. 9 is a diagram showing a multiple well information setting method for well images 7 in the second well displayed on the display unit 3. FIG. 9(A) to FIG. 9(D) show the display state of the well array images 70, which changes in response to operations. In FIG. 9, although all of the well images 7 shown in FIG. 3 are actually displayed on the display unit 3, only the minimum number of well images 7 necessary for explanation are shown due to the limitations of the drawing's range.

As shown in FIG. 9(A), it is assumed the case in which the well images 7 of "X1A," "X2A," "X3A," "X1B," "X2B," and "X3B," showing set images 77 each comprising a single set image 77a indicating that the well information has been set, and the welll images of "X1C," "X2C," and "X3C" with no well information set, are displayed in the second well of the well array image 70, the operator performs the following dragging operation on the display unit 3 in order to select the wells for which well information is to be additionally set. The assumed dragging operation is an operation in which the operator touches their finger to the display unit 3 and, for example, drags in the direction indicated by the arrow 71 in the figure from the well image 7 at "X1A" to the well image 7 at "X3C." This selects a region that includes the well images 7 corresponding to the setting target wells. In this example, in the six well images 7 where well information has already been set, all of them have the size standard sample set as their well information.

In response to the detection of the dragging operation shown in FIG. 9(A), the well images 7 in the second well, which fall within the range of the rows and columns covered by the dragging operation are selected. As a result, the actual wells corresponding to the well images 7 in the second well, which fall within the range of the rows and columns covered by the dragging operation, are selected as setting targets for well information. The well images 7 selected by the dragging operation are indicated as being highlighted, as indicated by the bold frames in the figure.

After performing the dragging operation shown in FIG. 9(A), when the operator releases their finger from the display unit 3, thereby releasing the touch state, the completion of the dragging operation is detected. When the end of the dragging operation is detected, as shown in FIG. 9(B), a third menu image 723 including a first selection option image 73, a second selection option image 74, and a third selection option image 76 is displayed. Note that the third menu image 723 can be displayed at any position as long as it is near the position where the dragging operation ended. As described above, by displaying the third menu image 723 near the position where the dragging operation ended, it becomes easier to visually recognize the third menu image 723 after the completion of the dragging operation.

As shown in FIG. 9(C), with the third menu image 723 displayed, when the operator performs, for example, a touch operation on the first selection option image 73, and the touch operation is detected, the selection option for the first selection option image 73 becomes selected. When the operation to select the selection option for the second selection option image 74 is performed, as illustrated in FIG. 9(C), data is stored in the storage unit 9. This data is used to set the "size standard" sample, corresponding to the first selection option image 73, as the sample to be injected into the wells corresponding to the well images 7, which were selected as setting targets for well information based on the dragging operation. Specifically, for the wells for which well information has already been set, data to be additionally set as samples for multiple injections is stored in the storage unit 9. For the wells for which well information has not been set yet, data to be set as samples for a single injection is stored in the storage unit 9.

As described above, with the third menu image 723 displayed, when the operator performs a touch operation on any one of the first selection option image 73 to the third selection option image 75, and once the touch operation is detected, the data is newly or additionally stored in the storage unit 9. The data is used to set the sample type corresponding to the touched selection option image as the sample to be injected into the wells corresponding to the well images 7 in the first well and the second well, which have been selected as the setting targets of the well information based on the dragging operation.

As shown in FIG. 9(C), with the third menu image 723 displayed, when the touch operation on the first selection option image 73 by the operator is detected, as shown in FIG. 9(D), the well image is displayed as follows. For the well images 7 for which well information has already been set, the set image 77a, consisting of a single set image 77a, changes to a multi-set image 77b in a display mode in which two circular images are superimposed to indicate that sample information for multiple injections has been set (hereinafter referred to as "multiple set"). The multi-set image 77b is displayed in a color different from the color of the sample shown in the first selection option image 73, the second selection option image 74, and the third selection option image 75. For the well images 7 for which well information has not been set yet, a single set image 77a, consisting of a circular image of the same color as that of the sample in the selection option image for which the touch operation was performed, is displayed. Furthermore, as shown in FIG. 9(D), the highlighting that was performed on the selected well image 7 is deactivated.

As described above, for the wells in the second well, it is possible to set the sample information for multiple injections. For the wells for which the sample information for multiple injections has been set, the set image 77 changes from the single set image 77a to the multi-set image 77b in the corresponding well images 7. As a result, it is possible for the operator to easily confirm which wells have been set with sample information for multiple injections. The setting of the sample information for multiple injections can be set, for example, up to a reference count of 120 injections for the sample information.

Note that the setting of the sample information for multiple injections may allow only the sample of the same type to be set in a single well, or samples of different types may be set in a single well. Note that the setting of the sample information for multiple injections may allow only the sample of the same type to be set in a single well, or samples of different types may be set in a single well.

The operation of setting the sample information for multiple injections for wells in the second well can also be performed based on selecting only the well images 7 corresponding to the well for which the sample information for one injection has been set based on a dragging operation. The operation of setting the sample information for multiple injections for wells in the second well can also be performed based on selecting only the well images 7 corresponding to the well for which the sample information for multiple injections has been set based on a dragging operation.

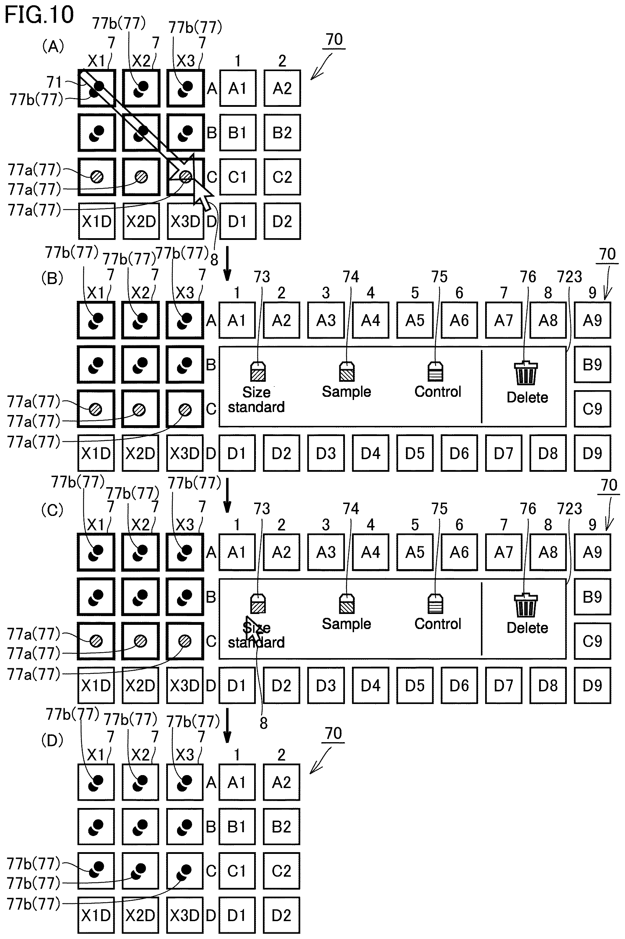

FIG. 10 is a diagram showing a multiple well information setting method for well images 7 in the second well displayed on the display unit 3. In FIG. 10, FIG. 10(A) to FIG. 10(D) show the display state of the well array image 70, which changes in response to operations. In FIG. 10, although all of the well images 7 shown in FIG. 3 are actually displayed on the display unit 3, only the minimum number of well images 7 necessary for explanation are shown due to the limitations of the drawing's range.

FIG. 10(A), in the state in which the well images 7 of well images 7 of "X1A", "X2A", "X3A", "X1B", "X2B", "X3B" with multi-set well information, and the well images 7 of "X1C", "X2C", and "X3C" with the "size standard" sample set as well information are displayed within the second well in the well array image 70, it is assumed that the operator performs the following dragging operation on the display unit 3 to select wells for setting information on a sample for multiple injections. In this example, in the six well images 7 in which well information has already been set, all of them have the size standard sample set as their well information.

In response to the detection of the dragging operation shown in FIG. 10(A), the well images 7 that fall within the range of the rows and columns covered by the dragging operation in the well array are selected. As a result, the actual wells corresponding to the well images 7 in the second well, which fall within the range of the rows and columns covered by the dragging operation, are selected as setting targets for well information. The well images 7 selected by the dragging operation are indicated as being highlighted, as shown by the bold frames in the figure.

After performing the dragging operation shown in FIG. 10(A), when the operator releases their finger from the display unit 3, thereby releasing the touch state, the completion of the dragging operation is detected. When the end of the dragging operation is detected, as shown in FIG. 10(B), a third menu image 723 including a first selection option image 73, a second selection option image 74, and a third selection option image 76 is displayed.

As shown in FIG. 10(C), with the third menu image 723 displayed, when the operator performs, for example, a touch operation on the first selection option image 73, and the touch operation is detected, the selection option for the first selection option image 73 becomes selected. When the operation to select the selection option for the first selection option image 73 is performed, as illustrated in FIG. 10(C), data is stored in the storage unit 9. This data is used to set the "size standard" sample, corresponding to the first selection option image 73, as the sample to be injected into the wells corresponding to the well images 7, which were selected as setting targets for well information based on the dragging operation. Specifically, for the wells for which multiple sets of well information have already been set, data to be additionally set as samples for multiple injections is stored in the storage unit 9. For the wells for which a single set of well information has been set, data to be set as samples for a single injection is stored in the storage unit 9.

As described above, with the third menu image 723 displayed, when the operator performs a touch operation on any one of the first selection option image 73 to the third option image 75, and once the touch operation is detected, the data is newly or additionally stored in the storage unit 9. The data is used to set the sample type corresponding to the touched selection option image as the sample to be injected into the wells corresponding to the well images 7 in the first well and the second well, which have been selected as the setting targets of the well information based on the dragging operation.

As shown in FIG. 10(C), with the third menu image 723 displayed, when the touch operation on the first selection option image 73 by the operator is detected, as shown in FIG. 10(D), the well image is displayed as follows. For the well images with multiple sets of well information, the images are maintained as the set images 77, specifically as the multi-set images 77b. For the well images 7 with a single set of well information, the set images 77 change to the multi-set images 77b, as shown in FIG. 10(D). Furthermore, as shown in FIG. 10(D), the highlighting that was performed on the selected well image 7 is deactivated.

### (Well Information Deletion Method for Well Images 7 in Second Well)

Next, a method for deleting well information in well images 7 in the second well will be described. One feature of the well information deletion method described below is as follows. That is, the operator selects a region in which both the well images 7 for which well information has been set and the well images 7 for which well information has not been set, in response to a dragging operation by touching their finger on the display unit 3. Thereafter, upon completion of the dragging operation by releasing their finger from the display unit 3, thereby ending the touch operation, the third menu image 723 is shown.

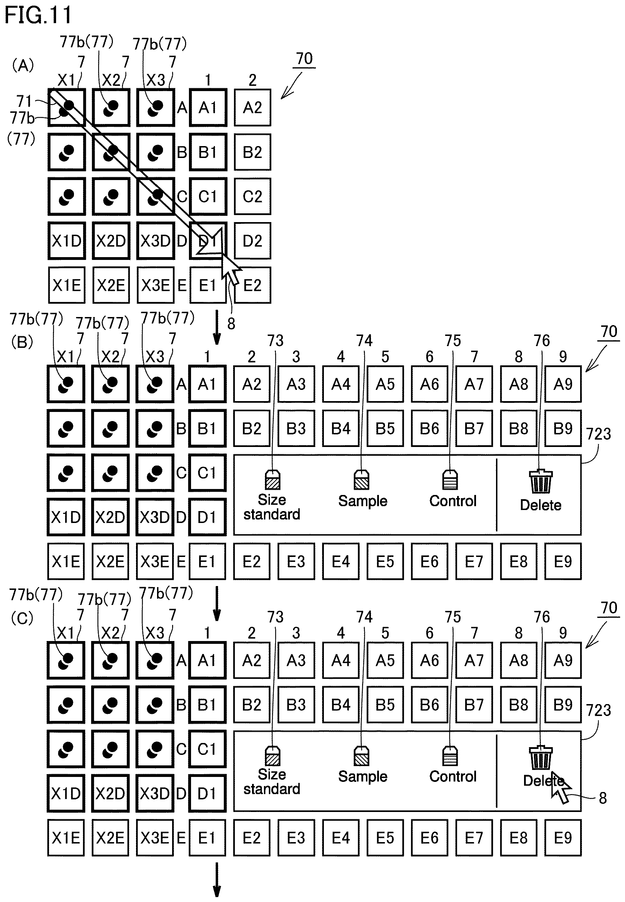

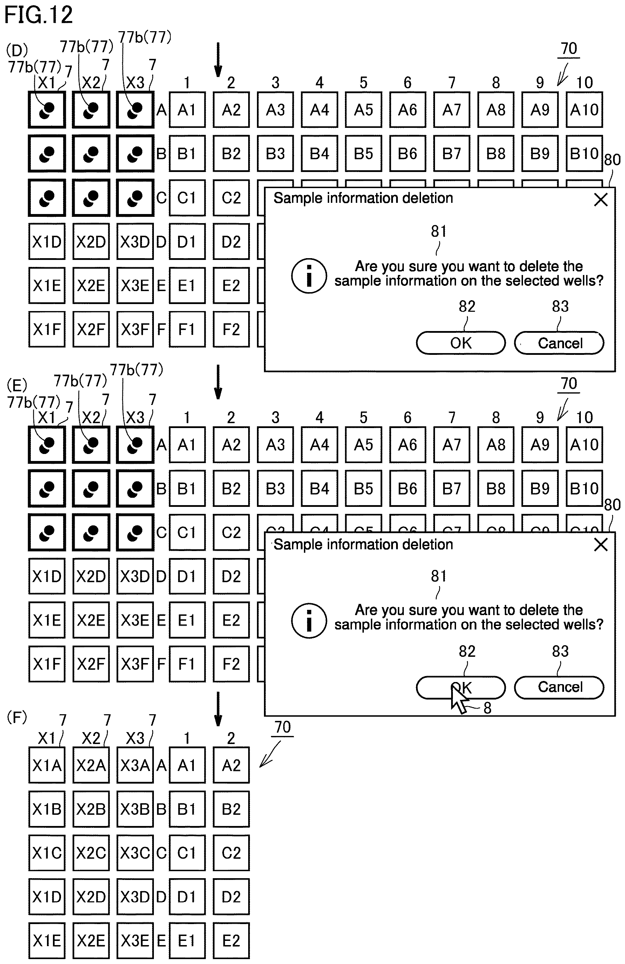

FIG. 11 and FIG. 12 show the well information deletion method for the well images 7 in the second well displayed on the display unit 3. In FIG. 11(A) to FIG. 11(C) and FIG. 12(D) to FIG. 12(F), the display state of the well array image 70, which changes in response to operations, is shown. In FIG. 11 and FIG. 12, although all of the well images 7 shown in FIG. 3 are actually displayed on the display unit 3, only the minimum number of well images 7 necessary for explanation are shown due to the limitations of the drawing's range.

As shown in FIG. 11(A), in the state in which in the nine well images 7 of "X1A" to "X3A," "X1B" to "X3B," and "X1C" to "X3C," a well array image 70 showing set images 77 consisting of multi-set images 77b indicating that the sample information for multiple injections has been set, it is assumed that the operator performs the following dragging operation on the display unit 3 in order to select wells in which well information is to be deleted. The assumed dragging operation is an operation in which the operator touches their finger to the display unit 3 and, for example, drags in the direction indicated by the arrow 71 in the figure from the well image 7 at "X1A" to the well image 7 at "D1." This selects a region that includes the well images 7 corresponding to the setting target wells.

In response to the detection of the dragging operation shown in FIG. 11(A), the well images 7 that fall within the range of the rows and columns covered by the dragging operation in the well array are selected. As a result, the actual wells corresponding to the well images 7 that include the well images 7 for which well information has been set and the well images 7 for which well information has not been set, which are included within the range of rows and columns where the dragging operation was performed, are selected. The well images 7 selected by such a dragging operation are indicated as being highlighted, as shown by the bold frames in the figure, allowing them to be distinguished from the well images 7 that have not been selected.

After performing the dragging operation shown in FIG. 11(A), when the operator releases their finger from the display unit 3, thereby releasing the touch state, the completion of the dragging operation is detected. When the completion of the dragging operation is detected, as shown in FIG. 11(B), a third menu image 723 including a deletion selection option image 76 is displayed on the right side, with the position from which the finger was released as the reference point.

As shown in FIG. 11(C), with the third menu image 723 displayed, when the operator performs a touch operation on the deletion selection option image 76 and the operation is detected, as shown in FIG. 12(D), a deletion confirmation image 80 will be displayed on the right side with the position from which the finger was released as a reference position in response to the touch operation.

As shown in FIG. 12(E), with the deletion confirmation image 80 displayed, when the operator touches the OK image 82 and the touch operation is detected, the well information stored in the storage unit 9 for the wells corresponding to the well images 7 for which well information has been set among the selected well images 7 is deleted according to the touch operation. Such a touch operation of the OK image 82 is also referred as a deletion confirmation operation. As shown in FIG. 12(F), in the well images 7 corresponding to the wells for which well information has been deleted out of the selected well images 7, the set images 77, which are composed of the multi-set image 77a, are deleted, and the well symbols are displayed. Further, as shown in FIG. 12(E), the highlighting that was displayed on the selected well images 7 is deactivated. This allows the operator to easily confirm the well images 7 corresponding to the wells for which well information has been deleted.

### (Well Selection Processing)

Next, the well selection processing executed by the controller 4 to select wells when setting and deleting well information as shown in FIG. 4 to FIG. 12 will be described by a flowchart.

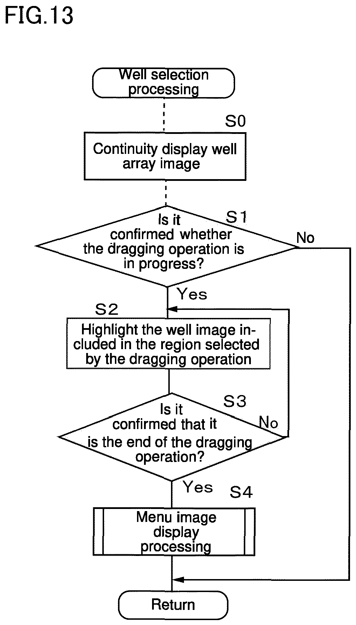

FIG. 13 is a flowchart showing the well selection processing. The well selection processing is performed by the CPU 41.

The CPU 41 performs the following processing for the well selection processing. In Step S (hereafter abbreviated as "S") 0, the well array image 70 is continuously displayed. In S 1, it is confirmed whether the dragging operation to select a region including well images corresponding to wells for which well information is to be set, as shown in FIG. 4, FIG. 5, FIG. 7 to FIG. 11, is in progress by the operator, based on the detection information on the touch operation from the operation detection unit of the touch panel 5.

In S1, if a dragging operation is not in progress, the routine returns. On the other hand, in S1, if a dragging operation is in progress, in S2, the wells included in the region selected by the dragging operation are confirmed, and the well images 7 corresponding to the selected wells are highlighted, as shown in FIG. 4, FIG. 5, FIG. 7 to FIG. 11. Next, in S3, it is confirmed whether it is the end of the dragging operation by determining whether the operator's finger has been released from the display unit 3 based on the detection information on the touch operation from the operation detection unit of the touch panel 5.

In S3, if it is confirmed that it is not the end of the dragging operation, the routine returns to S2. On the other hand, in S3, if it is confirmed that it is the end of the dragging operation, in S4, the menu image display processing is executed, and then the routine returns. The menu image display processing is the processing for displaying menu images such as the first menu image 721, the second menu image 722, and the third menu image 723 shown in FIG. 4, FIG. 5, and FIG. 7 to FIG. 11.

### (Menu Image Display Processing)

Next, the menu image display processing performed by the controller 4 to display menu images, such as the first menu image 721, the second menu image 722, and the third menu image 723, will be described by a flowchart.

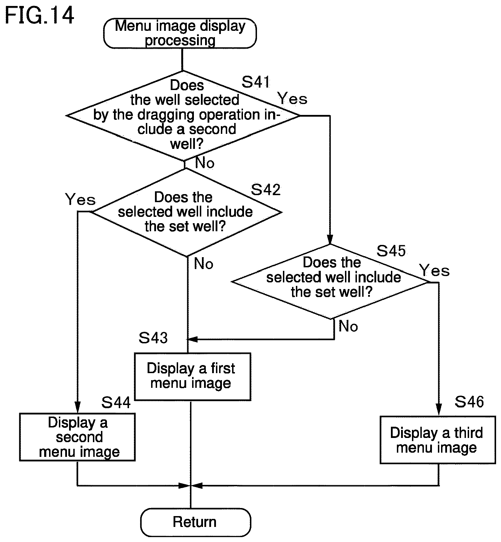

FIG. 14 is a flowchart showing the menu image display processing. The menu image display processing is executed by the CPU 41.

The CPU 41 performs the following processing in the menu image display processing. In S41, it is confirmed whether the wells selected by the dragging operation include the wells in the second well. If it is determined in S41 that the selected wells do not contain wells in the second well, i.e., the selected wells are only wells in the first well, then in S42 it is confirmed whether the selected wells include wells for which well information have already been set.

In S42, if it is determined that the selected wells do not include wells for which well information has already been set, i.e., the selected wells are only those for which well information has not been set, in S43, the first menu image 721 as shown in FIG. 4 is displayed, and then the routine returns. On the other hand, in S42, if it is determined that the selected wells include wells for which well information has already been set, in S44, the second menu image 722 as shown in FIG. 5 is displayed, and the routine returns.

Further, if it is determined that the wells selected in S41 include wells in the second well, in S45, it is confirmed whether the wells under selection include the wells for which well information has already been set.

In S45, if it is determined that the wells under selection do not include wells for which well information has already been set, i.e., the wells under selection are only those for which well information has not been set, in S43, the first menu image 721 as shown in FIG. 8 is displayed, and then the routine returns. On the other hand, in S45, if it is determined that the wells under selection include wells for which well information has already been set, in S46, the third menu image 723 as shown in FIG. 9 to FIG. 11 is displayed, and then the routine returns.

Note that in the case of executing the first well information overwrite processing in which the set well information on the first wells is overwritten with the information on the newly selected samples, instead of executing S44, processing for displaying a menu image such as the first menu image 721 is executed in S44A (not shown in the figure) to enable selection of the sample information to be overwritten.

### (Well Information Setting Processing)

Next, the well information setting processing for setting well information will be described by a flowchart based on the information on the sample selected in response to the selection operation of the option image in a menu image such as the first menu image 721, the second menu image 722, and the third menu image 723.

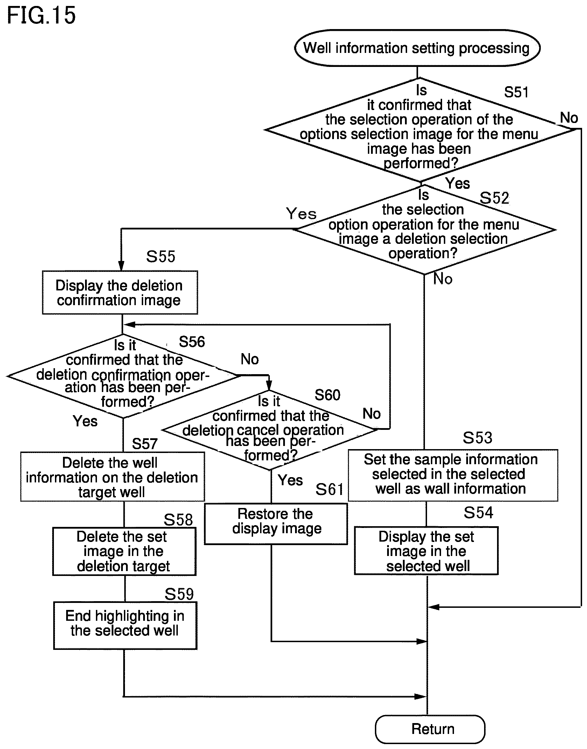

FIG. 15 is a flowchart showing the well information setting processing. The well information setting processing is performed by the CPU 41.

The CPU 41 executes the following processing in the well information setting processing. In S51, it is confirmed whether a selection operation for a selection option image for a menu image, such as the first menu image 721, the second menu image 722, and the third menu image 723, has been performed, based on the detection information on the touch operation from the operation detection unit of the touch panel 5. Specifically, the selection operation refers to a touch operation in which the operator selects the first selection option image 73, the second selection option image 74, the third selection option image 75, or the deletion selection option image 76.

In S51, if it is determined that no selection operation has been performed on the menu image, the routine returns. On the other hand, in S51, if it is determined that a selection operation for a selection option image on a menu image has been performed, in S52, it is confirmed whether the selection operation for the selection option image on the menu image is a deletion selection operation to select a deletion selection option image 76.

In S52, if it is determined that the selection operation for the selection option image on the menu image is not a deletion selection operation, that is, it is a sample selection operation to set a sample to be injected into wells by selecting the first selection option image 73, the second selection option image 74, or the third selection option image 75, in S53, data is stored in the storage unit 9. The data is for setting the information on the sample selected in response to the selection operation for the selection option image confirmed in S51 as the sample to be injected into the selected wells, corresponding to the wells selected by the well selection processing in FIG. 13. With this, the well information for the selected wells is set.

Next, in S54, set images 77 are displayed on well images 7 corresponding to the wells selected by the well selection processing in FIG. 13, based on the well information setting processing in S53, and the routine returns. In S54, if the well information indicating the set sample information is for a single injection, for example, a single set image 77a in a display color corresponding to the sample type, as shown in FIG. 4, is displayed as the set image 77. Further, in S54, if the well information indicating the set sample information is information indicating a sample for multiple injections, for example, a multi-set image 77b, as shown in FIG. 9, is displayed.

Further, in S52, if it is determined that the selection option for the selection option image on the menu image is a deletion selection operation, in S55, a deletion confirmation image 80 is displayed, as shown in FIG. 6 and FIG. 12. Next, in S56, it is confirmed whether a deletion confirmation operation in which the operator performs a touch operation on the OK image 82 in the deletion confirmation image 80 has been executed. When it is confirmed that the delete confirmation operation has been executed in S56, the well information, which is the sample information stored in the storage unit 9, is deleted for the wells whose well information is to be deleted in S57. Then, in S58, for the wells for which well information is to be deleted, the displayed set images 77 are deleted, as shown in FIG. 6 and FIG. 12. Next, as shown in FIG. 6 and FIG. 12, the highlighting performed for the wells selected by the well selection processing in FIG. 13 is terminated, and the routine returns.

On the other hand, in S56, if it is not confirmed that the delete confirmation operation was executed, in S60, it is confirmed whether the delete cancel operation, in which the operator performs the touch operation on the cancel image 83 in the deletion confirmation image 80, has been executed. In S60, if it is not confirmed that the delete cancel operation has been performed, the routine returns to S56. On the other hand, in S60, if it is confirmed that the delete cancel operation has been executed, in S61, the display state of the image is restored to the original state, and the routine returns. Specifically, in S61, the display state of the image is restored to the state before the execution of the dragging operation.

The well information setting information using the tablet terminal 1 is stored in the storage unit 9 of the tablet terminal 1, and is sent from the tablet terminal 1 to the sample injection device 10 when sample injection is performed by the sample injection device 10. Note that the well information set using the tablet terminal 1 may be sent to the storage device provided in the sample injection device 10 each time it is stored in the storage unit 9 of the tablet terminal 1. The same is true for the transmission of such well information in each of the following embodiments. The sample injection device 10 performs a sample injection operation for injecting the sample set for each well into each well based on the well information set using the tablet terminal 1.

Note that in the case of executing the first well information overwrite processing to overwrite the well information already set for the first well with the information on the newly selected sample, in S52, when it is determined that the selection operation for the selection option image on the menu image is not a deletion selection operation, it may be determined whether the first well information overwrite processing is being executed. If it is determined that first well information overwrite processing is being executed, the processing in S53A (not shown) to overwrite the already set well information with the information on the newly selected sample may be executed.

In the first embodiment described above, an example is shown in which multiple selection option images are displayed in the menu image, such as the first menu image 721 and the third menu image 723, but the present invention is not limited thereto. In other words, the selection option image to be displayed in the menu image may be at least one image.

In the first embodiment described above, in the array of a plurality of wells displayed by a plurality of well images 7, a menu image, such as the the first menu image 721 and the third menu image 723, which indicate, in a menu, at least one sample information that can be set in the wells corresponding to the well images 7 included in the region, in response to completion of the first operation of selecting, by a dragging operation, the region including the well image 7 to be set. With this, the dragging operation, which is the first operation, serves both as the selection operation of the region including the well images 7 to be set and the display operation of the second image that shows the information on the sample that can be set in the wells corresponding to the well images 7 in the menu. This can simplify the operation when setting the sample to be injected into the wells of the well plate.

### [Second Embodiment]

In the second embodiment, a well information setting method will be described. Here, the well plate 11 can be shared by multiple users. When the second user attempts to set well information for wells for which the first user has already set well information, the well image is displayed in a manner indicating that setting well information for these wells is not possible for the second user.

To manage the well plate 11 in which it can be shared by multiple users, it may be configured such that login information is assigned to each user, and when each user logs into the tablet terminal 1, the user can be identified based on the login information.

### [Well Information Setting Method]

### (Well Array Image)

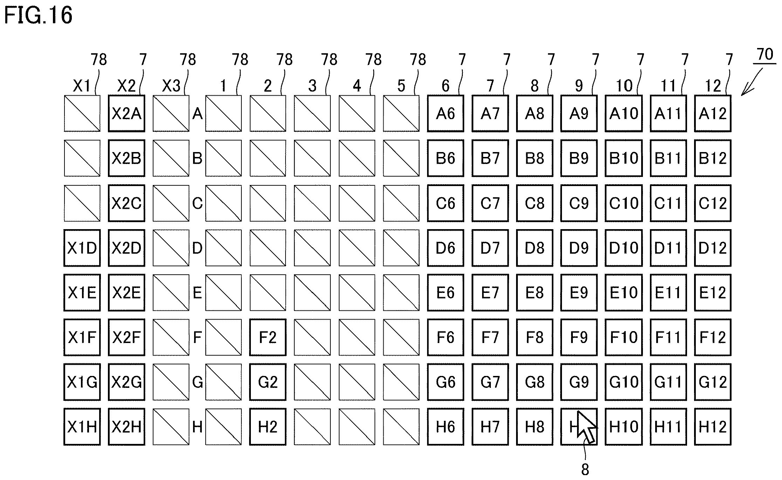

FIG. 16 is a diagram showing a well array image 70 displayed on a display unit 3 according to a second embodiment. The well array image 70 shown in FIG. 16 differs from the well array image 70 shown in FIG. 3 in that unusable well images 78 are displayed as well images. The unusable well image 78 is displayed as follows. For example, during the execution of an analysis on an analysis schedule using an analysis device, it is displayed based on the well information that had been stored before the analysis was paused.

The unusable well image 78 is displayed to indicate that the well is unusable so that, assuming that the well plate 11 can be shared by multiple users, when a second user sets well information for a well for which well information has already been set by the first user, it becomes impossible for the second user to set well information. In the following second embodiment, portions that differ from the first embodiment will be mainly described.

Unlike the well image 7, the unusable well image 78 is displayed in such a manner as to remind the viewer that the well is an unusable well, such as not showing the well symbol, being lower in image density than the well image 7, or showing a diagonal line.

The unusable well images 78 as described above are displayed as follows. For example, the unusable well images 78 are displayed based on the well information that had been stored prior to the pause during the execution of the analysis for the wells on the well sheet in the schedule of an analysis using an analysis device. Specifically, the unusable well images 78 are displayed as follows. Analysis is performed by the analysis device according to the analysis schedule. The analysis is then paused. At that pause, when editing the well information on the well sheet to be analyzed, the wells whose well information had been stored prior to the pause are displayed as unusable well images 78. Further, when adding a well sheet to be analyzed, the wells for which well information had been stored prior to the pause are also displayed as unusable well images 78.

### (Well Information Setting Method in Well Images 7 in First Well and Second Well)

Next, the well information setting method for the well images 7 in the first well will be described. One feature of the well information setting method described below is as follows. That is, an operator selects a region displaying well images 7 with unset well information by touching and dragging their finger on the display unit 3. Upon releasing the finger from the display unit 3, thereby ending the series of drag operations, a first menu image 721 is displayed. This first menu image 721 shows selection options for the sample to be set in the wells within the selected region.

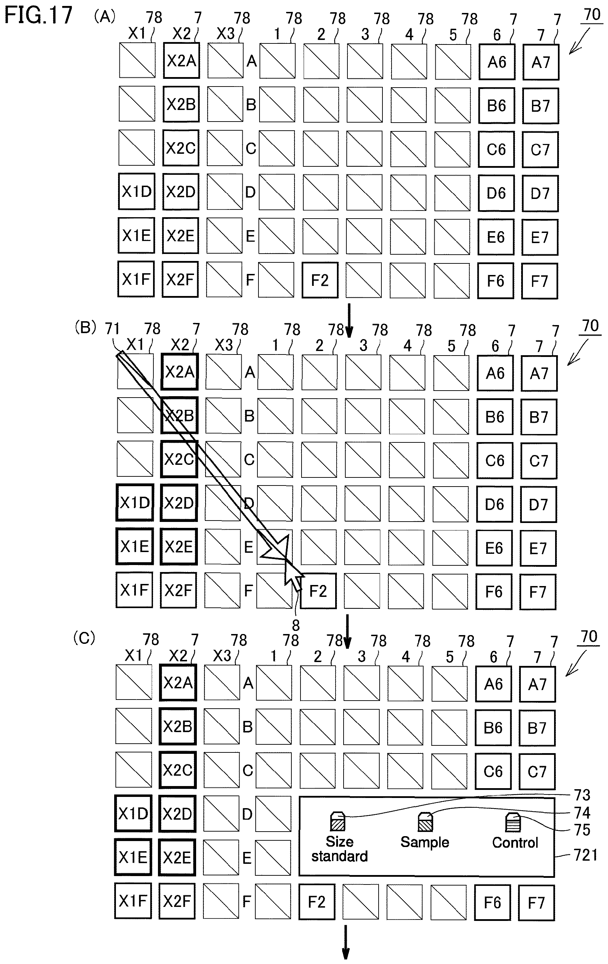

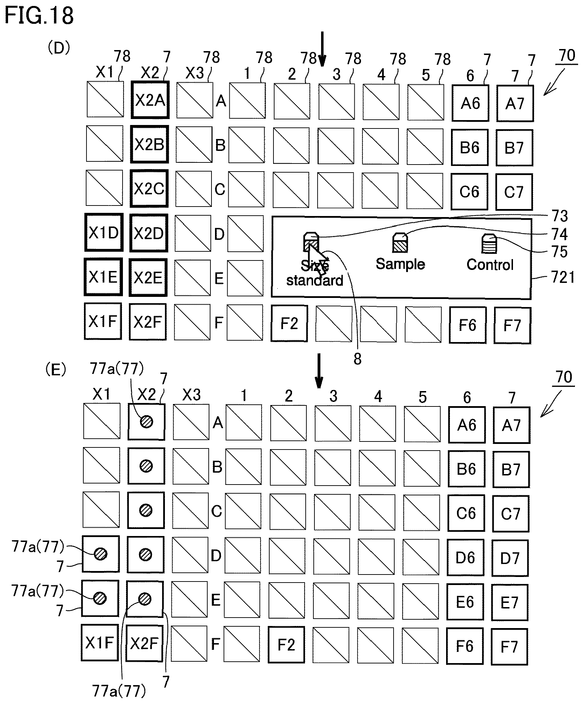

FIG. 17 and FIG. 18 show the well information setting method for well images 7 in the first well and the second well displayed on the display unit 3. In FIG. 17 and FIG. 18, the display state of the well array image 70, which changes in response to the display operation, is shown in FIG. 17(A) to FIG. 17(C) and FIG. 18(D) and FIG. 18(E). In FIG. 17 and FIG. 18, although all of the well images 7 shown in FIG. 16 are actually displayed on the display unit 3, only the minimum number of well images 7 necessary for explanation and unusable well images 78 are shown due to the limitations of the drawing's range.

As shown in FIG. 17(A), it is assumed that an operator performs the following dragging operation on the display unit 3 to select wells for setting well information in the first and second well information in a state in which the well array image 70 in which the well images for which wall information has not been set and unusable well images 78 are displayed. As shown in FIG. 17(B), the assumed dragging operation is an operation in which the operator touches their finger to the display unit 3 and, for example, drags in the direction indicated by the arrow 71 in the figure from the unusable well image 78 at "X1A" to the unusable well image 78 at "E1." This selects a region that includes the well images 7 corresponding to the setting target wells in the first well and the second well.

In response to the detection of the dragging operation shown in FIG. 17(B), the well images 7 that fall within the range of the rows and columns covered by the dragging operation in the well array are selected. The unusable well images 78 included within the range of rows and columns covered by the dragging operation are not selected. As a result, the actual wells corresponding to the well images 7 within the range of the rows and columns covered by the dragging operation are selected as setting targets for well information. Such a dragging operation is also referred to as a well selection operation. The well images 7 selected by such a dragging operation are indicated as being highlighted, as shown by the bold frames in the figure, allowing them to be distinguished from the well images 7 that have not been selected.

After performing the dragging operation shown in FIG. 17(B), when the operator releases their finger from the display unit 3, thereby releasing the touch state, the completion of the dragging operation is detected. When the completion of the dragging operation is detected, as shown in FIG. 17(C), a first menu image 721 is displayed on the right side, with the position from which the finger was released as the reference point.

As shown in FIG. 18(D), with the first menu image 721 displayed, when the operator performs, for example, a touch operation on the first selection option image 73, and the touch operation is detected, the selection option of the first selection option image 73 becomes selected. When the operation to select the selection option for the first selection option image 73 is performed, as illustrated in FIG. 18(D), data is stored in the storage unit 9. This data is used to set the "size standard" sample, corresponding to the first selection option image 73, as the sample to be injected into the wells corresponding to the well image 7, which were selected as targets for well information settings based on the aforementioned dragging operation.

As described above, with the first menu image 721 displayed, when the operator performs a touch operation on any one of the first selection option image 73 to the third selection option image 75, and once the touch operation is detected, the data is stored in the storage unit 9. The data is used to set the sample type corresponding to the touched selection option image as the sample to be injected into the wells corresponding to the well images 7, which has been selected as the setting target of the well information based on the dragging operation.

As shown in FIG. 18(D), when a touch operation of the first selection option image 73 by the operator, for example, is detected while the first menu image 721 is displayed, single set images 77a, each consisting of a circular image of the same color as the sample image color in the touched selection option image, are displayed as the set images 77 in the well image 7 selected as the targets of well information setting. Furthermore, as shown in FIG. 18(E), the highlighting that was performed on the selected well image 7 is deactivated. This allows the operator to easily confirm which type of sample has been set in which well, since the set type of sample is indicated by images in the selected well images 7.

Further, for the unusable well images 78, even if they exist within the designated range for the dragging operation, they are not selected as the targets for setting well information and no well information is set. Therefore, the possibility that the first user changes the well information on the wells for which well information has been set by the second user is suppressed.

### (Well Information Deletion Method for Well Images 7 in Second Well)

Next, a method for deleting well information in well images 7 in the second well will be described. One feature of the well information deletion method described below is as follows. That is, the operator selects a region in which both the well images 7 for which well information has been set and the well images 7 for which well information has not been set, in response to a dragging operation by touching their finger on the display unit 3. Thereafter, upon completion of the dragging operation by releasing their finger from the display unit 3, thereby ending the touch operation, the third menu image 723 is shown.

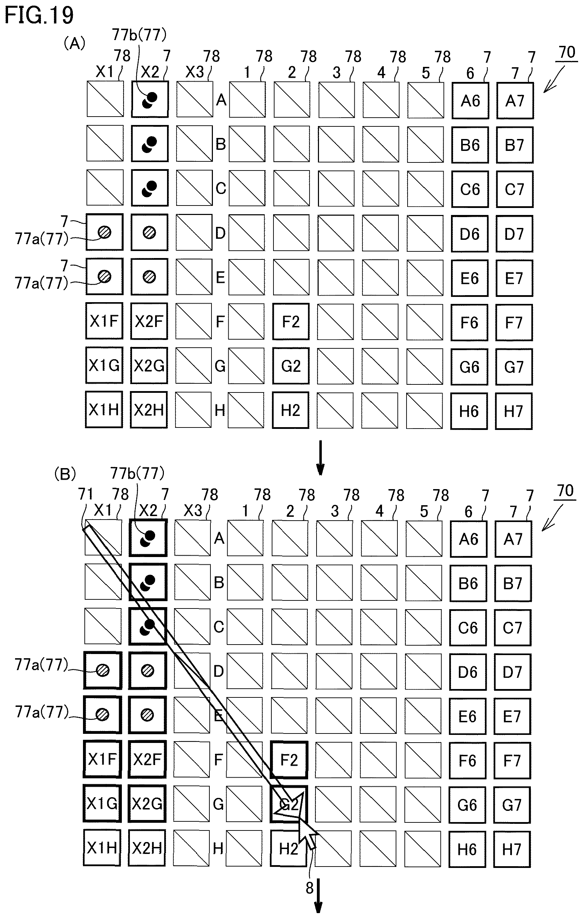

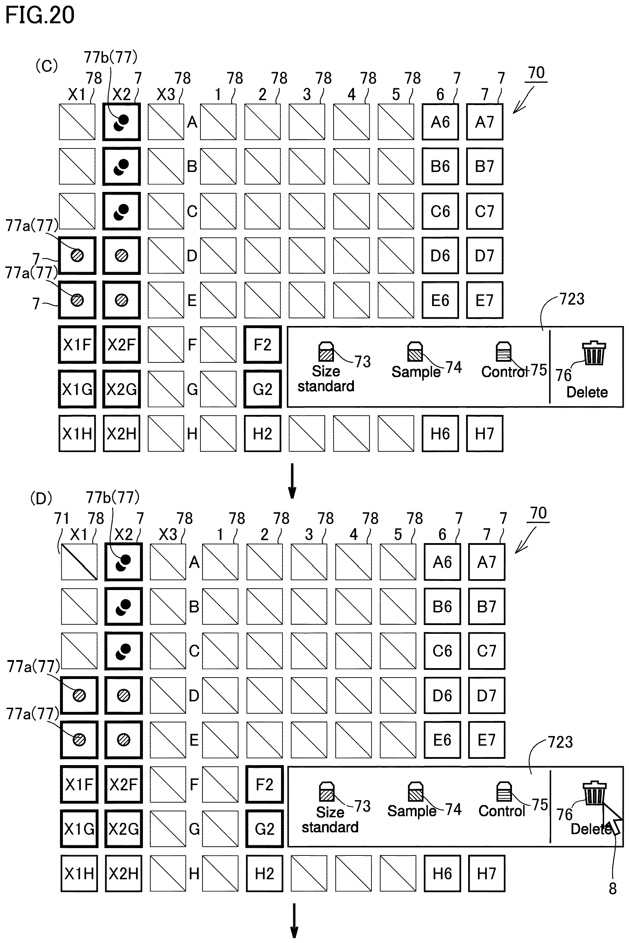

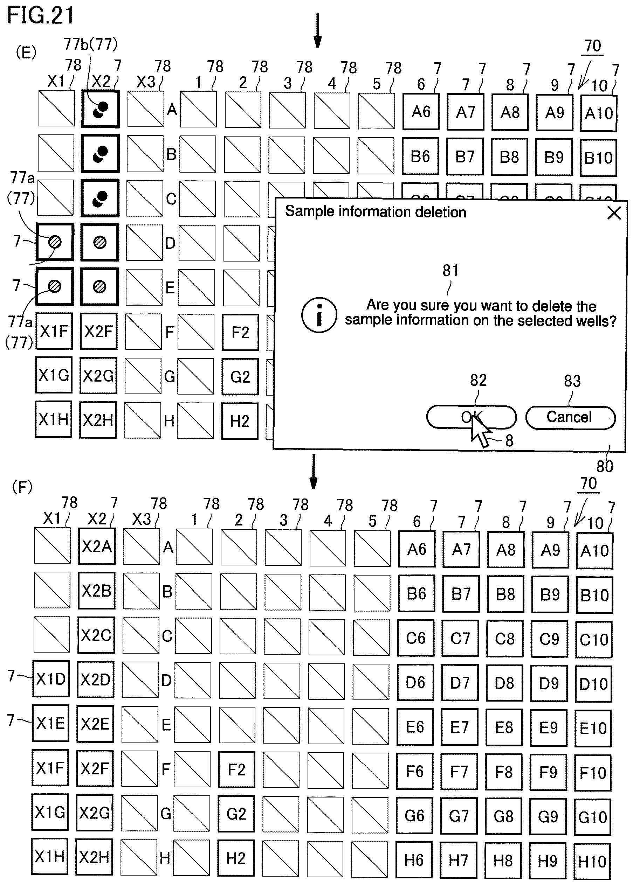

FIG. 20 and FIG. 21 show the well information deletion method for the well images 7 in the second well displayed on the display unit 3. In FIG. 19(A) and (B), FIG. 20(C) and (D), and FIG. 21(E) and (F), the display state of the well array image 70, which changes in response to the operation, is shown. In FIG. 19 to FIG. 21, although all of the well images 7 shown in FIG. 16 are actually displayed on the display unit 3, only the minimum number of well images 7 necessary for explanation are shown due to the limitations of the drawing's range.

As shown in FIG. 19(B), it is assumed that an operator performs the following dragging operation on the display unit 3 to select wells for which well information is to be deleted in a state in which the well array image 70 is displayed. The well array image 70 depicts set images 77 comprising single set images 77a, indicating that the well information has been set or set images 77 comprising multi-set images 77, in 7 (seven) well images of "X1D," and "X1E," "X2A" to "X2E." The assumed dragging operation is an operation in which the operator touches their finger to the display unit 3 and, for example, drags in the direction indicated by the arrow 71 in the figure from the well image 7 at "X1A" to the well image 7 at "G2." This selects a region that includes the well images 7 corresponding to the deletion target wells.

In response to the detection of the dragging operation shown in FIG. 19(B), the well images 7 that fall within the range of the rows and columns covered by the dragging operation in the well array are selected. As a result, the actual wells corresponding to the well images 7 that include the well images 7 for which well information has been set and the well images 7 for which well information has not been set, except for the unusable well images 78, within the range of rows and columns covered by the dragging operation, are selected. The well images 7 selected by the dragging operation are indicated as being highlighted, as shown by the bold frames in the figure, allowing them to be distinguished from the well images 7 that have not been selected.

After performing the dragging operation shown in FIG. 19(B), when the operator releases their finger from the display unit 3, thereby releasing the touch state, the completion of the dragging operation is detected. When the end of the dragging operation is detected, as shown in FIG. 20(C), the third menu image 723 including the deletion selection option image 76 is displayed on the right side of the screen, with the position where the finger was released as a reference position.

As shown in FIG. 20(D), with the third menu image 723 displayed, when the operator performs a touch operation on the deletion selection option image 76 and the operation is detected, as shown in FIG. 21(E), a deletion confirmation image 80 will be displayed on the right side with the position from which the finger was released as a reference position in response to the touch operation.

Various processing such as adding well information and deleting well information in the second embodiment can be performed basically in the same manner as in the first embodiment.

The well information setting method shown in the second embodiment can be realized by executing the same processing performed by the controller 4 shown in FIG. 13 to FIG. 15 of the first embodiment. In that case, the well array image 70, as shown in FIG. 16, will be displayed in the processing in S0 shown in FIG. 13. Further, the display to change well images 7 to unusable well images 78 is performed each time the user changes, by checking the well information set by the other user.

As shown in FIG. 21(E), with the deletion confirmation image 80 displayed, when the operator touches the OK image 82 and the touch operation is detected, the well information stored in the storage unit 9 for the wells corresponding to the well images 7 for which well information has been set among the selected well images 7 is deleted according to the touch operation. Such a touch operation of the OK image 82 is also referred as a deletion confirmation operation. As shown in FIG. 21(F), in the well images 7 corresponding to the wells for which well information has been deleted out of the selected well images 7, the set images 77, which are composed of the single set images 77a or multi-set images 77b indicating that the well information has been set, are deleted and the well symbol is displayed. Further, as shown in FIG. 21(F), the highlighting that was displayed on the selected well images 7 is deactivated. This allows the operator to easily confirm the well images 7 corresponding to the wells for which well information has been deleted.

In the second embodiment described above, in the array of a plurality of wells displayed by a plurality of well images 7, a menu image, such as the the first menu image 721 and the third menu image 723, which indicate, in a menu, at least one sample information that can be set in the well corresponding to the well images 7 included in the region, in response to completion of the first operation of selecting, by dragging operation, the region including the well image 7 to be set. With this, the dragging operation, which is the first operation, serves both as the selection operation of the region including the well images 7 to be set and the display operation of the second image that shows the information on the sample that can be set in the wells corresponding to the well images 7 in the menu. This can simplify the operation when setting the sample to be injected into the wells of the well plate.

In addition, the unusable well images 78 are selected as targets for deletion of well information, even if they exist in the designated range of the dragging operation, so the risk of the first user deleting the well information of a well for which the second user has set well information is suppressed.

### [Third Embodiment]

Next, in the third embodiment, the well information setting method in the case of using a part of the first well of the well plate 11 as a dilution well will be described.

### [Well Information Setting Method]

### (Well Array Image)

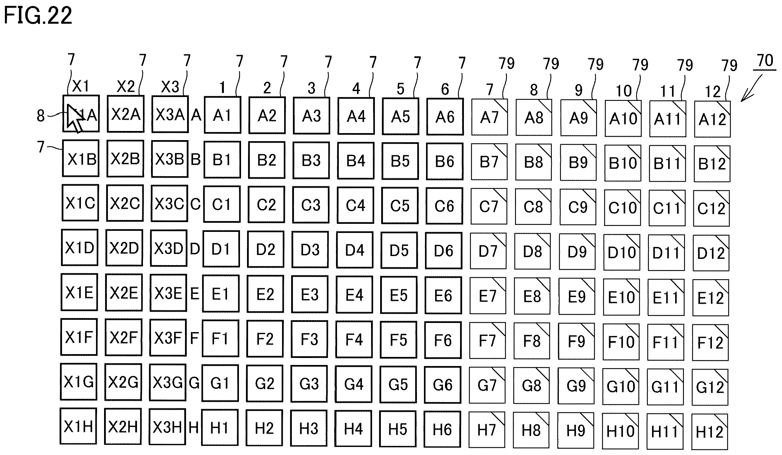

FIG. 22 shows a well array image 70 displayed on the display unit 3 according to the third embodiment.

The well array image 70 shown in FIG. 22 differs from the well array image 70 shown in FIG. 3 in that dilution well images 79 are displayed as well images. In the first well, the wells in rows 1 to 6 are for injecting a sample at the reference concentration, and the wells in rows 7 to 12 are for injecting a sample at a concentration diluted beyond the reference concentration.

Each of the dilution wells in row 7 is injected with a sample diluted from the same sample that is injected into each of the wells in row 1. Each of the dilution wells in row 8 is injected with a sample diluted from the same sample that is injected into each of the wells in row 2. Each of the dilution wells in row 9 is injected with a sample diluted from the same sample that is injected into each of the wells in row 3. Each of the dilution wells in row 10 is injected with a sample diluted from the same sample that is injected into each of the wells in row 4. Each of the dilution wells in row 11 is injected with a sample diluted from the same sample that is injected into each of the wells in row 5. Each of the dilution wells in row 12 is injected with a sample diluted from the same sample that is injected into each of the wells in row 6.

The well array image 70 in FIG. 22 differs from the well array image 70 in FIG. 3 in that the wells in rows 7 to 12 are displayed as dilution well images 79, indicating dilution wells. The dilution well images 79 are lower in density than the well images 7, and a triangle is shown in part of the image to indicate that it is a dilution well image.

### (Well Information Setting Method for Well Images 7 in First Well)

Next, the well information setting method in the well images 7 in the first well will be described. One feature of the well information setting method described below is as follows. That is, an operator selects a region displaying well images 7 with unset well information by touching and dragging their finger on the display unit 3. Upon releasing the finger from the display unit 3, thereby ending the series of drag operations, a first menu image 721 is displayed. This first menu image 721 shows selection options for the samples to be set in the wells within the selected region.

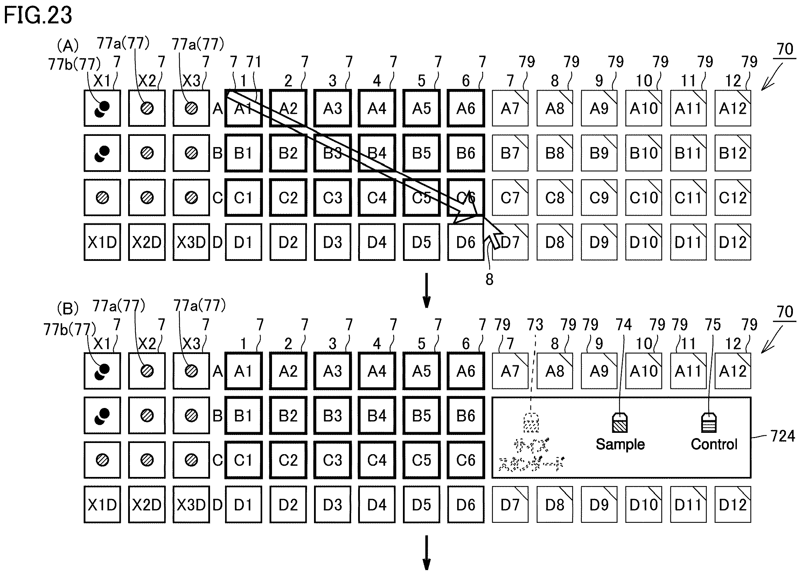

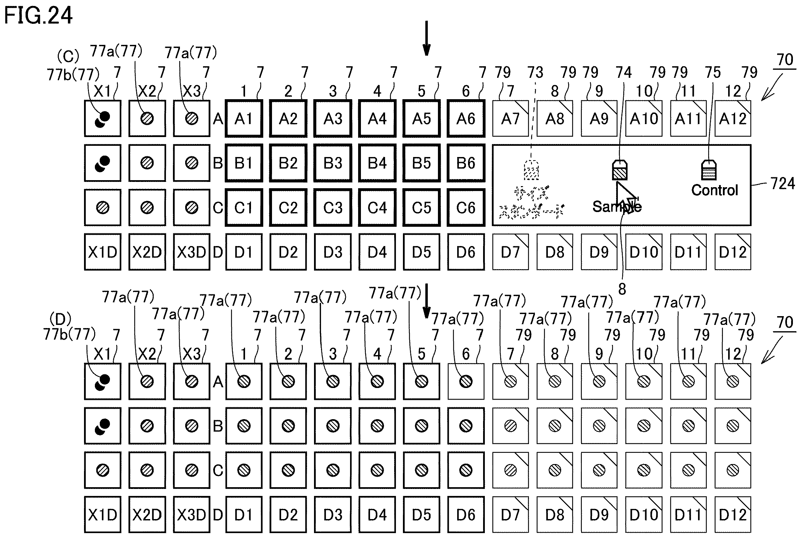

FIG. 23 and FIG. 24 show the well information setting method for the well images 7 in the first well displayed on the display unit 3. In FIG. FIG. 23 and FIG. 24, the display state of the well array images 70, which change in response to operations, is shown in FIG. 23(A) and (B), and FIG. 24(C) and (D). In FIG. 23 and FIG. 24, although all of the well images 7 and dilution well images shown in FIG. 22 are actually displayed on the display unit 3, only the minimum number of well images 7 and dilution well images 79 necessary for explanation are shown due to the limitations of the drawing's range.

As shown in FIG. 23(A), it is assumed that an operator performs the following dragging operation on the display unit 3 to select wells for setting well information in a state in which all of the wells in the first well have not been set with well information. The assumed dragging operation is an operation in which the operator touches their finger to the display unit 3 and, for example, drags in the direction indicated by arrow 71 in the figure from the well image 7 at "A1" to the well image 7 at C6." This selects a region that includes the well images 7 corresponding to the setting target wells.

In response to the detection of the dragging operation shown in FIG. 23(A), the well images 7 that fall within the range of the rows and columns covered by the dragging operation in the well array are selected. As a result, the actual wells corresponding to the well images 7 within the range of the rows and columns covered by the dragging operation are selected as setting targets for well information. The well images 7 selected by such a dragging operation are indicated as being highlighted, as shown by the bold frames in the figure, allowing them to be distinguished from the well images 7 that have not been selected.

After performing the dragging operation shown in FIG. 23(A), when the operator releases their finger from the display unit 3, thereby releasing the touch state, the completion of the dragging operation is detected. When the end of the dragging operation is detected, as shown in FIG. 23(B), a fourth menu image 724 including a first selection option image 73, a second selection option image 74, and a third selection option image 75 is displayed. The fourth menu image 724 indicates that the first selection option image 73 cannot be selected as an option by being displayed in a darker image than the other selection option images. Note that the fourth menu image 724 can be displayed at any position as long as it is near the position where the dragging operation ended. As described above, since the fourth menu image 724 is displayed near the position where the dragging operation ended, it becomes easier to visually recognize the fourth menu image 724 after the completion of the dragging operation.

As shown in FIG. 24(C), with the fourth menu image 724 displayed, when the operator performs, for example, a touch operation on the second selection option image 74, and the touch operation is detected, the selection option of the second selection option image 74 becomes selected. As shown in FIG. 24(C), when the operation to select the selection option of the second selection option image 74 is performed, the sample labeled "Sample" corresponding to the second selection option image 74, data is stored in the storage unit 9. The data is used to set the sample to be injected into the wells corresponding to the well image 7 selected as the well information setting target based on the aforementioned dragging operation, as the sample to be injected into the wells corresponding to the well images 7 selected as the setting target of the well information based on the dragging operation, and the sample to be injected to the dilution wells corresponding to the dilution well image 79 that has a correspondence relation with the selected well image 7.

As described above, with the fourth menu image 724 displayed, when the operator performs a touch operation on the second selection option image 74 or the third selection option image 75, and once the touch operation is detected, the data is stored in the storage unit 9. The data is used to set the sample type corresponding to the touched selection option image as the sample to be injected into the wells corresponding to the well images 7, which has been selected, as the setting target of the well information based on the dragging operation and the dilution well corresponding to the selected well images 7.

As shown in FIG. 24(C), when a touch operation of the second selection option image 74 by the operator, for example, is detected while the fourth menu image 724 is displayed, as shown in FIG. 24(D), single set images 77a, each comprising a circular image of the same color as the sample image color in the touched selection option image, are displayed as the set images 77 in the well images 7 selected as the targets of well information setting. As shown in FIG. 24(D), single set images 77a, each comprising a circular image of the same color as the image color of the sample in the selection option image for which the touch operation was performed, are displayed as the set images 77 in the dilution well images 79 that are in a correspondence relation with the well images 7 that were selected as the well information setting targets. The color of the single set image 77a, which is displayed as the set image 77 in the dilution well image 79, is displayed in a lower density color than the color of the set image 77 displayed in the well image 7. Further, as shown in FIG. 24(D), the highlighting that was performed on the selected well images 7 is deactivated.

As described above, with the fourth menu image 724 displayed, when the operator performs a touch operation on any one of the first selection option image 73 to the third selection option image 75, and once the touch operation is detected, the data is stored in the storage unit 9. The data is used to set the sample type corresponding to the touched selection option image as the sample to be injected into the wells corresponding to the well images 7, which have been selected as the setting targets of the well information based on the dragging operation and the dilution well corresponding to the selected well images 7.

Various processing such as adding well information and deleting well information in the third embodiment can be performed basically in the same manner as in the first embodiment. Note that in the third embodiment, the first menu image 721 and the third menu image 723 may be displayed instead of the fourth menu image 724.

The well information setting method shown in the third embodiment can be realized by executing the same processing performed by the controller 4 shown in FIG. 13 to FIG. 15 of the first embodiment. In that case, the well array image 70, as shown in FIG. 22, will be displayed in the processing in S0 shown in FIG. 13.

As shown in FIG. 24(C), when the operator performs a touch operation, for example, on the second selection option image 74 with the first menu image 721 displayed, and the touch operation is detected, as shown in FIG. 24(D), set images 77 which are single set images each composed of a circular image of the same color as the image color of the sample in the operated selected option image, are displayed in the well images 7 selected as the well information setting targets based on the aforementioned dragging operation, and in the dilution well images 79 corresponding to the well images 7. Further, as shown in FIG. 24(D), the highlighting that was performed on the selected well images 7 is deactivated. As a result, the type of the sample set in the selected well image 7 and the types of samples set in the corresponding dilution well images 79 are displayed by means of images, so that the operator can easily confirm which type of the sample was set in which well.

In the third embodiment described above, in the array of a plurality of wells displayed by a plurality of well images 7, a menu image, such as the fourth menu image 24, which indicate, in a menu, at least one sample information that can be set in the well corresponding to the well images 7 included in the region, in response to completion of the first operation of selecting, by dragging operation, the region including the well image 7 to be set. With this, the dragging operation, which is the first operation, serves both as the selection operation of the region including the well images 7 to be set and the display operation of the menu image that shows the information on the sample that can be set in the wells corresponding to the well images 7 in the menu. This can simplify the operation when setting the sample to be injected into the wells of the well plate.

In addition, when a part of the first wells of the well plate 11 can be used as dilution wells, a dilution well image 79 is automatically assigned as the well image, thereby simplifying the operation when setting up samples to be injected into the wells of the well plate including the dilution well.

### [Modified Embodiments]

(1) The communication between the tablet terminal 1 and the sample injection device 10 may be performed by either wireless or wired communication.
(2) Instead of the tablet terminal 1, a computer, such as a notebook personal computer in which a dragging operation is executed with a mouse, may be used. In other words, the sample information setting method for setting sample information in an injection device that injects a sample into each of the plurality of wells in a well plate as described above, in response to an operator's operation, can also be implemented by displaying various images on a display device, such as a liquid crystal display device that is not of the touch panel type, and executed based on the operator's manipulation of an input device such as a mouse.
(3) The set image 77 shown in the well image 7 may indicate the type of the sample that has been set by using an image that indicates only color, only shape, or a combination of both color and shape.
(4) When the set image 77 is shown in the well image 7, the well symbol may not be displayed, or the well symbol may be displayed.
(5) For setting the sample to be injected using well images 7 in the first well, it may be possible to set the sample to be injected multiple times for a single well image 7.
(6) It may be configured such that the first menu image 721 and the second menu image 722 are commonly used, a menu image, capable of displaying a first selection option image 73, a second selection option image 74, a third selection option image 75, and a deletion selection option image 76, is displayed, when the deletion selection option image 76 cannot be selected, an indication that deletion selection option image 76 cannot be selected is displayed, and when the first selection option image 73, the second selection option image 74, and the third selection option image 75 cannot be selected, an indication that these images cannot be selected is displayed.
(7) When displaying the multi-set image 77b as the set image 77, the multi-set image 77b may be displayed in a display mode that identifies the type of sample set to be injected multiple times by color or other means.
(8) When setting sample information using the well image 7 and the first menu image 721 or the third menu image 723, an image of an input field displaying a comment may be displayed, allowing the operator to enter a comment or other comment text regarding the sample to be set. In such cases, comments can be entered for information that cannot be fully expressed only by the name of the sample, such as experimental conditions.
(9) The well information set using the tablet terminal 1 may be stored in a storage unit provided in the sample injection device 10, in addition to being stored in the storage unit 9 of the tablet terminal 1.
(10) The well information set using the tablet terminal 1 may be stored in a storage unit provided in the sample injection device 10, in addition to being stored in the storage unit 9 of the tablet terminal 1.
(11) The well information set using the tablet terminal 1 may be stored only in the storage unit 9 of the tablet terminal 1 and sent from the tablet terminal 1 to the sample injection device 10 when sample injection is performed by the sample injection device 10.
(12) The highlighting of a well image, such as the well image 7, may be achieved by making the display color of the well image to be highlighted darker than the reference intensity, or by making the shape of the well image to be highlighted different from the reference shape.
(13) The first well and the second well may be displayed in different colors to clarify the difference in the type of wells by using different colors in the well image, and the shape of the well image may be displayed in different shapes to clarify the difference in the type of wells.
(14) The unusable well image 78 may be visually distinguishable from the usable well image 7 by making the color intensity of the image lighter than the well image 7, or it may be visually distinguishable from the usable well image 7 by having a different shape than the well image 7.
(15) The dilution well image 79 may be visually distinguishable from the usable well image 7 by making the color intensity of the image lighter than the well image 7, or it may be visually distinguishable from the usable well image 7 by having a different shape than the well image 7.
(16) In the display unit 3, an image showing the set results of the well information using the well image 7, etc., of the well array image 70 in a tabular format as described above may be displayed on the same screen as the screen on which the well array image 70 is displayed. By doing so, it is possible to confirm the set state of the well information both in the well array image 70 and in the tabular image.
(17) When deleting the well information by selecting the well images 7 corresponding to the wells for which well information has already been set, the condition may be set such that only the well images 7 for which well information has already been set are selected.
(18) In the first embodiment to the third embodiment, a sample information setting method is described in which the sample information is set in an injection device for injecting a sample into each of a plurality of wells provided in a well plate in response to an operator's operation. However, not limited to this, the techniques related to the sample information setting method described in the first to third embodiments may be applied to a sample information setting method for setting sample information in response to an operator's operation in an injection device that injects a sample into each of multiple vials in a vial rack. A vial rack is a rack in which multiple injection target containers, such as vials, can be placed, and is used, for example, in a liquid chromatograph and a gas chromatograph, among other auto-samplers. In other words, the aforementioned embodiments need only be a sample information setting method for setting sample information in response to an operator's operation in an injection device that injects a sample into each of a plurality of injection targets.

### [Appendix]

The sample information setting method of this disclosure has the following features.
(1) A sample information setting method of setting sample information according to an operator's operation in an injection device (sample injection device 10) for injecting a sample into each of a plurality of injection targets (wells), the method comprising:
   a step of displaying a plurality of first images (well images 7) corresponding to the plurality of injection targets, respectively (SO);
   a step of displaying a second image (first menu image 721, third menu image 723) showing, as a menu, information (first selection option image 73, second selection option image 74, third selection option image 75) on at least one sample capable of being set to the injection target (well) corresponding to the first image (well image 7) included in a region within an array of the plurality of injection targets displayed by the plurality of first images, in response to completion of a first operation (dragging operation) of selecting the region, the region including the first images (well images) of the injection target (well) (S4); and
   a step of setting information on the sample selected by a second operation (selection option operation) from the information on the at least one sample included in the second image (first menu image 721, third menu image 723) to the injection target corresponding to the first images included in the region (S53).
   According to this configuration, in response to the completion of the first operation of selecting a region including the first images to be set by the dragging operation in an array of multiple injection targets displayed by multiple first images, at least one of the injection targets that can be set for the injection targets corresponding to the first images included in that region in response to the completion of the first operation of selecting a region including the first image to be set and displaying a second menu image showing information on the sample information that can be set for the injection target corresponding to the first image. Thus, the first operation serves as the selection operation of the region including the first image to be set and the display operation of the second menu image showing information on the sample that can be set for the injection target corresponding to the first image. Therefore, it is possible to simplify the operations for setting multiple samples to be injected. Further, the sample information that can be set for the injection targets corresponding to the first images included in the region selected by the first operation is displayed in menu by the second image. The sample information selected by the second operation from the sample information included in the second image is set for the injection targets corresponding to the first images included in the region. Therefore, it is possible to simplify the task of applying the same setting to multiple injection targets.
(2) The information on the sample is information on a type of the sample to be injected into each of the plurality of injection targets (the type of sample, such as, "Size Standard," "Sample," and "Control"). According to this configuration, since the information on the sample is information about the type of sample to be injected into each of the plurality of injection targets, it is possible to simplify the operation of setting the type of sample to be injected into each of the plurality of injection targets.
(3) The second image (first menu image 721, third menu image 723) is displayed near a position where the first operation ended. According to such a configuration, since the second image is displayed near the position where the first operation ended, it becomes possible to easily recognize the second image after the end of the first operation.
(4) The first image (well image) and the second image (first menu image 721, third menu image 723) are displayed on a display device (the display unit of a touch panel type, or a non-touch panel type display device), and the first operation ends in response to completion of a series of operations (completion of a series of dragging operation) when the dragging operation is performed.
   According to this configuration, since the first operation ends in response to the completion of the series of operations when the dragging operation is performed, when the second image (first menu image 721, third menu image 723) is displayed in response to the completion of the first operation, operations other than the first operation can be performed to simplify the operator's operation. Since the second image can be displayed without further execution, the operator's operation can be simplified.
(5) The method further includes a step of displaying the first images (well images 7) included in the region in a different display manner (color of the set image 77) depending on the sample selected by the second operation (selection option selection operation) (S54).
   According to this configuration, the first image included in the region is displayed in a different display mode according to the sample selected by the second operation, so that information on the sample set as the injection target can be easily confirmed visually.
(6) The method further includes:
   a step of displaying an item (deletion selection option image 76) to delete information on the sample that has already been set in a menu of the second image (second menu image 722, third menu image 723) when the first operation is performed on the first images (wells) corresponding to the injection target (well) for which the sample to be injected has been set (S4); and
   a step of deleting the information on the sample that has already been set when the item to delete the information is selected by the second operation (selection option selection operation) (S57).
   According to this configuration, when the item to be deleted is selected by the second operation, the information for the sample that has already been set is deleted, making it possible to remove already set sample information as necessary.
(7) Each of the plurality of injection targets is capable of setting the information on the sample to be injected for a number of injections, and the method further comprises a step of displaying the first image in a different manner (the display manner of a single set image 77a and the display manner of multi-set image 77b are different from each other) according to a number of injections of the sample, based on the information on the sample set for the injection target (second well) (S54).
   According to this configuration, the first image is displayed in a different manner depending on the set number of injections, making it easy to visually confirm the set number of injections.
(8) The plurality of injection targets includes a first injection target (first well) and a second injection target (second well), the first injection target (first well) is capable of setting the information on the sample to be injected by a single injection number, the second injection target (second well) is capable of setting the information on the sample to be injected by a plurality of injection numbers, and
   the method further comprises
   a step of changing the information on the sample that has already been set with the information on the sample selected by the second operation in a form of overwriting when setting the information on the sample to be injected to the first injection target (first well) (S44A and S53A, not illustrated),
   a step of adding information on the sample selected by the second operation to the information on the sample that has already been set when setting the information on the sample to be injected to the second injection targets (S53).
   According to this configuration, when setting the information of the sample to be injected for the first injection target, the sample information that has already been set is changed in the form of overwriting the information of the sample selected by the second operation. Therefore, it is possible to easily change the type of sample to be set for the first injection target for which the sample information has already been set.
(9) The plurality of injection targets (wells) is capable of being shared by a plurality of users including a first user and a second user, and
   the method further comprises a step of indicating that it is impossible for the first user to set the information on the sample for the first images to which the information on the sample has already been set (indicating an unusable well image 78) by the second user (S0 used in the second embodiment).
   According to this configuration, when the first user sets the sample information, it is indicated by the second user that the first image for which the sample information is set cannot be set, and thus the possibility of the first user changing or deleting the sample information in the wells for which the sample information is set is suppressed. Therefore, the possibility of the first user changing or deleting the sample information in the wells where the sample information has been set can be suppressed.
(10) The plurality of injection targets is a plurality of wells (first well, second well) in a well plate (well plate 11). According to this configuration, it is possible to simplify the operation when setting samples to be injected into multiple wells.
   The sample information setting program of this disclosure has the following features.
(11) A sample information setting program for making a computer execute processing to set information on a sample according to an operator's operation in an injection device (sample injection device 10) for injecting a sample into each of a plurality of injection targets (wells), the processing comprising:
   a step of displaying a plurality of first images (well images 7) corresponding to the plurality of injection targets (wells), respectively (S0);
   a step of displaying a second image (first menu image 721, third menu image 723)) showing, as a menu, information (first selection option image 73, second selection option image 74, third selection option image 75) on at least one sample capable of being set to the injection target corresponding to the first images included in a region within an array of the plurality of injection targets displayed by the plurality of first images (well images), in response to completion of a first operation (dragging operation) of selecting the region, the region including the first images of the injection targets (S4); and
   a step of setting information on the sample selected by a second operation (selection option selection operation) from the information on the at least one sample included in the second image (first menu image 721, third menu image 723) to the injection targets corresponding to the first images included in the region (S53).
   According to this configuration, upon the completion of the first operation, which involves selecting a region that includes a target first image via a dragging operation within an array of multiple injection targets displayed by multiple first images, a second image is displayed. This second image shows a menu of at least one sample's information that can be set for the injection target corresponding to the first image within that region. Therefore, the first operation serves dual purposes: it is both the act of selecting a region containing the target first image and the act of displaying the second image, which presents the information of a sample that can be set for the injection target corresponding to that first image. This setup simplifies the process of setting samples to be injected into multiple injection targets. In addition, the second menu image shows the sample information that can be set for the injection target corresponding to the first image included in the region selected by the first operation, and the sample information selected by the second operation from the sample information included in the second image is set for the injection target corresponding to the first image included in the region. This approach simplifies the task of applying the same settings to multiple injection targets.
(12) The plurality of injection targets is a plurality of wells (first well, second well) in a well plate (well plate 11). According to this configuration, it is possible to simplify the operation when setting samples to be injected into multiple wells.
   The sample information setting device of this disclosure has the following features.
(13) A sample information setting device (tablet terminal 1) for setting information on a sample according to an operator's operation in an injection device (sample injection device 10) for injecting a sample into each of a plurality of injection targets (wells), the device comprising:
   a display unit (display unit 3);
   a controller (controller 4); and
   a storage unit (storage unit 9),
   wherein the controller (controller 4) executes
   first image display processing for displaying a plurality of first images (well images) corresponding to a plurality of injection targets (wells), respectively, in the display unit (S0), and
   second image display processing for displaying a second image (first menu image 721, third menu image 723) showing, as a menu, information on at least one sample capable of being set to the injection target corresponding to the first image (well image) included in a region within an array of the plurality of injection targets displayed by the plurality of first images (well images), in response to completion of a first operation (dragging operation) of selecting the region, the region including the first images of the injection targets (wells) (S4), and
   setting processing for causing the storage unit to store data for setting the information on the sample selected by a second operation from the information on the at least one sample included in the second image (first menu image 721, third menu image 723) to the injection targets (wells) corresponding to the first images included in the region (S53).
   According to this configuration, in response to the completion of the first operation of selecting a region including the first image to be set by the dragging operation in an array of multiple injection targets displayed by multiple first images, the second image showing a menu of the information on at least one of samples capable of being set for the injection corresponding to the first image included in the region is displayed, and the first operation also serves as the selection operation of the region including the first image to be set and the display operation of the second image showing information on the sample that can be set for the injection target corresponding to the first image, thereby simplifying the operations for setting multiple samples to be injected. In addition, the second menu image shows the sample information that can be set for the injection target corresponding to the first image included in the region selected by the first operation, and the sample information selected by the second operation from the sample information included in the second image is set for the injection target corresponding to the first image included in the region. This approach simplifies the task of applying the same settings to multiple injection targets.
(14) The plurality of injection targets is a plurality of wells (first well, second well) in a well plate (well plate 11). According to this configuration, it is possible to simplify the operation when setting samples to be injected into multiple wells.

Note that the embodiments disclosed here should be considered illustrative and not restrictive in all respects. It should be noted that the scope of the invention is indicated by claims and is intended to include all modifications within the meaning and scope of the claims and equivalents.

### Description of Reference Symbols

- 10:: Sample injection device
- 7:: Well image
- 70:: Well array image
- 721:: First menu image
- 1:: Tablet terminal
- 4:: Controller
- 41:: CPU
- 9:: Storage unit
- 78:: Unusable well image
- 79:: Dilution well image

## Claims

1. A sample information setting method of setting information on a sample according to an operator's operation in an injection device for injecting a sample into each of a plurality of injection targets, the method comprising:
a step of displaying a plurality of first images corresponding to the plurality of injection targets, respectively;
a step of displaying a second image showing, as a menu, information on at least one sample capable of being set to the injection target corresponding to the first image included in a region within an array of the plurality of injection targets displayed by the plurality of the first images, in response to completion of a first operation of selecting the region by a dragging operation, the region including the first image of the injection target; and
a step of setting the information on the sample selected by a second operation from the information on the at least one sample included in the second image to the injection target corresponding to the first image included in the region.

2. The sample information setting method as recited in claim 1,
wherein the information on the sample is information on a type of sample to be injected into each of the plurality of injection targets.

3. The sample information setting method as recited in claim 1 or 2,
wherein the second image is displayed near a position where the first operation ended.

4. The sample information setting method as recited in claim 1,
wherein the first image and the second image are displayed on a display device, and
wherein the first operation ends in response to completion of a series of operations when the dragging operation is performed.

5. The sample information setting method as recited in claim 1, further comprising:
a step of displaying the first image included in the region in a different display manner depending on the sample selected by the second operation.

6. The sample information setting method as recited in claim 1, further comprising:
a step of displaying an item to delete the information on the sample that has already been set in the menu of the second image when the first operation is performed on the first image corresponding to the injection target for which the sample to be injected has already been set; and
a step of deleting the information on the sample that has already been set when the item to delete the information is selected by the second operation.

7. The sample information setting method as recited in claim 1,
wherein each of the plurality of injection targets is capable of setting the information on the sample to be injected for a number of injections, and
wherein the method further comprises a step of displaying the first image in a different manner according to a number of injections of the sample, based on the information on the sample set for the injection target.

8. The sample information setting method as recited in claim 1,
wherein the plurality of injection targets includes a first injection target and a second injection target,
wherein the first injection target is capable of setting the information on the sample to be injected for a single injection,
wherein the second injection target is capable of setting the information on the sample to be injected for a plurality of injections, and
wherein the method further comprises
a step of changing the information on the sample that has already been set with the information on the sample selected by the second operation in a form of overwriting when setting the information on the sample to be injected to the first injection target, and
a step of adding information on the sample selected by the second operation to the information on the sample that has already been set when setting the information on the sample to be injected to the second injection target.

9. The sample information setting method as recited in claim 1,
wherein the plurality of injection targets is capable of being shared by a plurality of users including a first user and a second user, and
wherein the method further comprises a step of indicating that it is impossible for the first user to set the information on the sample for the first image to which the information on the sample has already been set by the second user.

10. The sample information setting method as recited in claim 1,
wherein the plurality of injection targets is a plurality of wells provided in a well plate.

11. A sample information setting program for making a computer execute processing to set information on a sample according to an operator's operation in an injection device for injecting a sample into each of a plurality of injection targets, the processing comprising:
a step of displaying a plurality of first images corresponding to the plurality of injection targets, respectively;
a step of displaying a second image showing, as a menu, information on at least one sample capable of being set to the injection target corresponding to the first image included in a region within an array of the plurality of injection targets displayed by the plurality of the first images, in response to completion of a first operation of selecting the region by a dragging operation, the region including the first image of the injection target; and
a step of setting information on the sample selected by a second operation from the information on the at least one sample included in the second image to the injection target corresponding to the first image included in the region.

12. The sample information setting program as recited in claim 11,
wherein the plurality of injection targets is a plurality of wells provided in a well plate.

13. A sample information setting device for setting information on a sample according to an operator's operation in an injection device for injecting a sample into each of a plurality of injection targets, the device comprising:
a display unit;
a controller; and
a storage unit,
wherein the controller executes
first image display processing for displaying a plurality of first images corresponding to the plurality of injection targets, respectively, on the display unit,
second image display processing for displaying a second image showing, as a menu, information on at least one sample capable of being set to the injection target corresponding to the first image included in a region within an array of the plurality of injection targets displayed by the plurality of the first images, in response to completion of a first operation of selecting the region by a dragging operation, the region including the first image of the injection target, and
setting processing for causing the storage unit to store data for setting the information on the sample selected by a second operation from the information on the at least one sample included in the second image to the injection target corresponding to the first image included in the region.

14. The sample information setting device as recited in claim 13,
wherein the plurality of injection targets is a plurality of wells provided in a well plate.
